# EUROPÄISCHE PATENTSCHRIFT

(11) **EP 3 307 235 B1**
(45) Veröffentlichungstag und Bekanntmachung des Hinweises auf die Patenterteilung: **07.04.2021**
(21) Anmeldenummer: 16723035.8
(22) Anmeldetag: 26.04.2016
(51) Int. Cl.: A61K 8/46, A61Q 5/08

(54) **EMULSIONEN ZUR VERBESSERTEN REDUKTIVEN ENTFÄRBUNG KERATINISCHER FASERN**
BETTER DECOLORATION OF KERATINIC FIBERS
DÉCOLORATION AMÉLIORÉE POUR DES FIBRES KÉRATINIQUES

(30) Priorität: 12.06.2015 DE 102015210751
(43) Veröffentlichungstag der Anmeldung: 18.04.2018
(73) Patentinhaber: Henkel AG & Co. KGaA, 40589 Düsseldorf (DE)
(72) Erfinder: SCHÖPGENS, Jürgen, 41366 Schwalmtal (DE); MÜLLER, Burkhard, 40221 Düsseldorf (DE)
(86) Internationale Anmeldenummer: PCT/EP2016/059227
(87) Internationale Veröffentlichungsnummer: WO 2016/198203

(56) Entgegenhaltungen:
- WO-A2-2007/107310
- WO-A2-2007/107312
- DE-A1- 19 629 453
- DE-A1-102006 022 274
- DE-A1-102006 053 343
- DE-A1-102006 053 402
- "Colorants and Finishing Products", , 1. Januar 2000 (2000-01-01), XP055232024, Gefunden im Internet: URL:http://www2.basf.us/pc_textiles/pdfs/C yclanonECO.pdf [gefunden am 2015-11-27]

## Beschreibung

Die vorliegende Erfindung betrifft kosmetische Mittel zur reduktiven Entfärbung gefärbter keratinischer Fasern, welche mindestens ein Sulfinsäurederivat sowie mindestens einen Alkohol mit 10 bis 30 Kohlenstoffatomen enthalten. Weiterhin betrifft die vorliegende Erfindung eine Verpackungseinheit (Kit-of-parts), ein Verfahren zur reduktiven Entfärbung von gefärbten keratinischen Fasern sowie die Verwendung der erfindungsgemäßen kosmetischen Mittel und der erfindungsgemäßen Verpackungseinheit zur reduktiven Entfärbung sowie zur Verringerung der Geruchsbelastung währen der reduktiven Entfärbung gefärbter keratinischer Fasern.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Weiterhin ist eine Haarfärbung unter Verwendung von Vorstufen des natürlichen Haarfarbstoffs Melanin möglich. Nach Aufbringung dieser Vorstufen erfolgt im Haar die Ausbildung naturanaloger Farbstoffe im Rahmen oxidativer Prozesse. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Bei den zuvor angeführten Färbeverfahren kann es jedoch vorkommen, dass die erzielte Färbung, insbesondere Haarfärbung, aus verschiedenen Gründen ganz oder teilweise wieder rückgängig gemacht werden soll. So kann dies vom Verbraucher beispielsweise gewünscht sein, wenn das Färbeergebnis auf den Fasern dunkler ausfällt oder nicht der gewünschte Farbton erzielt worden ist. Weiterhin kann eine Entfärbung von gefärbten Fasern erforderlich sein, wenn die Fasern, insbesondere Haare, nur für einen konkreten Anlass in einer bestimmten Nuance gefärbt oder getönt werden und nach einigen Tagen die ursprüngliche Haarfarbe wieder hergestellt werden soll.

Mittel und Verfahren zur Entfärbung gefärbter Fasern, insbesondere Haare, sind im Stand der Technik bekannt. So kann beispielsweise durch eine oxidative Nachbehandlung der gefärbten Haare mithilfe eines üblichen Blondiermittels eine Entfärbung gefärbter Haare erreicht werden. Jedoch tritt bei diesem Verfahren aufgrund des Einsatzes starker Oxidationsmittel eine Schädigung der Haare auf.

Ferner sind auch reduktive Prozesse zur Entfärbung gefärbter Fasern, insbesondere Haare, bekannt. Die reduktive Entfärbung wird hierbei durch Reduktion der auf den Keratinfasern bzw. Haaren befindlichen Farbstoffe statt erreicht. Bei diesem Vorgang werden die in den Farbstoffen vorhandenen Doppelbindungen reduziert, das chromophore System der Farbstoffe auf diese Weise unterbrochen und der Farbstoff in eine farblose Form, auch als reduzierte Leukoform bezeichnet, umgewandelt.

So offenbart die europäische Patentanmeldung EP 1 300 136 A2 beispielsweise ein Verfahren zur Haarbehandlung, bei welchem die Haare zunächst gefärbt und anschließend reduktiv entfärbt werden. Die reduktive Entfärbung wird in diesem Verfahren durch Verwendung eines Dithionitsalzes in Kombination mit einem Tensid erreicht. In der WO 2008/055756 A2 wird die reduktive Entfärbung von Keratinfasern mittels eines Gemisches aus einem Reduktionsmittel und einem Absorptionsmittel beschrieben. In der WO2007/107312 wird das Natriumsalz der 2-Hydroxy-2-sulfinoessigsäure als Entfärbemittel verwendet.

Ein generelles Problem bei den aus dem Stand der Technik bekannten reduktiven Entfärbemitteln besteht darin, dass die gefärbten Keratinfasern durch Einsatz des Reduktionsmittels zwar entfärbt werden können, diese Entfärbung jedoch nicht lange anhält. Insbesondere bei oxidativ gefärbten Haaren, bei welchen die Färbung durch Oxidationsfarbstoffvorprodukte vom Entwickler- und vom Kupplertyp erzeugt wird, werden Färbungen mit teilweise sehr guten Echtheitseigenschaften erhalten. Bei Anwendung der reduktiven Entfärbemittel werden diese Farbstoffe zwar reduktiv in ungefärbte Verbindungen überführt, diese ungefärbten Verbindungen verbleiben jedoch aufgrund der guten Echtheitseigenschaften auch nach der reduktiven Entfärbung im Haar. Unter Einwirkung von Luftsauerstoff können diese ungefärbten Verbindungen zurückoxidiert werden, wodurch eine Rückfärbung stattfindet. Diese Rückfärbung entspricht in der Regel nicht dem Farbton der vor der Entfärbung vorgenommenen Färbung, sondern kann äußerst unattraktiv ausfallen und ist daher vom Anwender des reduktiven Entfärbemittels unerwünscht. Weiterhin ist die Geruchsbelastung bei Anwendung dieser Entfärbemittel hoch und wird daher vom Verbraucher als unangenehm empfunden.

Aufgabe der vorliegenden Erfindung war daher die Bereitstellung eines kosmetischen Mittels zur reduktiven Entfärbung von gefärbten keratinischen Fasern, welches in einer möglichst vollständigen und lang anhaltenden Entfärbung der gefärbten Fasern resultiert. Insbesondere auf zuvor mit oxidativen Färbemitteln auf Basis von Oxidationsfarbstoffvorprodukten vom Entwickler- und vom Kupplertyp gefärbten Fasern soll das kosmetische Mittel eine gute Entfärbeleistung aufweisen. Weiterhin sollen nach der Entfärbung unter der Einwirkung von Luftsauerstoff keine Rückfärbungen, Nuancenverschiebungen und/oder Nachdunklungen auftreten. Zudem soll bei Anwendung dieser Mittel die Geruchsbelastung minimiert werden. Schließlich soll das kosmetische Mittel toxikologisch unbedenklich sein und sich unter kosmetisch unbedenklichen Bedingungen anwenden lassen.

Es wurde nun überraschenderweise gefunden, dass die Verwendung von bestimmten Sulfinsäuren in Kombination mit speziellen Alkoholen zu sehr guten Entfärbeleistungen auf oxidativ gefärbten Fasern führt und keine Rückfärbungen, Nuancenverschiebungen oder Nachdunklungen auftreten. Eine besonders gute Entfärbeleistung ohne Rückfärbungen und Nachdunklungen wird hierbei auf zuvor mit oxidativen Färbemitteln gefärbten Fasern erreicht. Weiterhin führt der Einsatz der zuvor genannten Kombination zu einer äußerst geringen Geruchsbelastung während der Anwendung. Die in den erfindungsgemäßen kosmetischen Mitteln eingesetzten Verbindungen sind toxikologisch unbedenklich und lassen sich unter Bedingungen anwenden, welche nicht zu einer übermäßigen Schädigung der Haare und der Kopfhaut führen.

Ein erster Gegenstand der Erfindung ist daher ein kosmetisches Mittel zur reduktiven Entfärbung von gefärbten keratinischen Fasern, insbesondere menschlichen Haaren, enthaltend in einem kosmetischen Träger
a) mindestens eine Verbindung I der Formel (I)

   A[(CR¹R²)SO₂M]_{p,q} (I)

   in welcher
   - A: für N(R³)_{3-q} oder O(R⁴)₂₋ₚ steht
   - R¹, R², R⁴: unabhängig voneinander für ein Wasserstoffatom oder für eine C₁-C₆-Alkylgruppe stehen,
   - R³: für identische oder verschiedene Reste ausgewählt aus einem Wasserstoffatom, einer C₁-C₂₀-Alkylgruppe, einer C₃-C₈-Cycloalkylgruppe ggf. substituiert durch ein bis drei C₁-C₄-Alkylreste steht
   - M: für identische oder verschiedene Reste ausgewählt aus einem Wasserstoffatom oder einem Äquivalent eines Alkali-, Erdalkali- oder Metallions, insbesondere für Natrium, Kalium, ½ Magnesium, ½ Calcium, ½ Zink, oder für ein Ammoniumion (NH₄⁺) steht,
   - p: für die Zahlen 1 oder 2 steht,
   - q: für die Zahlen 1, 2 oder 3 steht,
   wobei
   - mindestens einer der Reste R¹, R², R⁴ für eine C₁-C₆-Alkylgruppe steht, wenn A für O(R⁴₂₋ₚ) steht, und
   - R³ nicht für ein Wasserstoffatom steht, wenn q gleich 1 ist, und
b) mindestens einen Alkohol mit 10 bis 30 Kohlenstoffatomen in einer Gesamtmenge von 0,1 bis 20 Gew.-%, bezogen auf das Gesamtgewicht des kosmetischen Mittels, dadurch gekennzeichnet, dass es mindestens eine Verbindung I der Formel (la) N(CH₂SO₂Na)₃ enthält.

Verfahren zur reduktiven Nachbehandlung von Polyester-Textilien mit Sulfinsäurederivaten der Formel (I) sind beispielsweise aus EP 0 914 516 B1 bekannt. Bei der Nachbehandlung der Polyester-Textilien werden die Sulfinsäure-Derivate der Formel (I) jedoch ausschließlich unter kosmetisch wenig akzeptablen Bedingungen bei Temperaturen von 50 bis 100 °C eingesetzt. Es wurde nun überraschenderweise gefunden, dass sich diese Sulfinsäurederivate auch zur Entfärbung von keratinischen Fasern bzw. menschlichen Haaren eignen, wenn die keratinischen Fasern zuvor mit direktziehenden Farbstoffen und/oder Oxidationsfarbstoffvorprodukten (Entwicklern und Kupplern) gefärbt wurden. Insbesondere überraschend war in diesem Zusammenhang, dass die reduktive Entfärbung der Keratinfasern bereits unter physiologisch akzeptablen Bedingungen, d.h. bei Temperaturen unterhalb von 45 °C, erfolgt.

Die Substituenten R¹ bis R⁴ der Verbindung I der Formel (I) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Beispiele für C₇-C₂₀-Alkylgruppen sind die Gruppen n-Octyl, n-Decyl, n-Dodecyl, n-Tetradecyl, n-Hexadecyl und n-Octadecyl. Beispiele für eine C₃-C₆-Cycloalkylgruppe sind die Cyclopropylgruppen Cyclopentylgruppen oder Cyclohexylgruppen. Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin werden unter dem Begriff "gefärbte keratinische Fasern" Keratinfasern verstanden, welche mit herkömmlichen, dem Fachmann bekannten kosmetischen Färbemitteln gefärbt wurden. Insbesondere sind unter den "gefärbten keratinischen Fasern" Fasern zu verstehen, welche mit den aus dem Stand der Technik bekannten oxidativen Färbemitteln (Entwicklern und Kupplern) gefärbt wurden. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, welche das entsprechende Wissen des Fachmannes wiedergeben

Zudem werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen erfindungsgemäßen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels - sofern nichts anderes angegeben ist - auf das Gesamtgewicht des anwendungsbereiten Entfärbemittels, d.h. des Mittels, welches zur Anwendung bereit ist und direkt vom Anwender auf die zuvor gefärbten keratinischen Fasern appliziert werden kann.

Bei den erfindungsgemäßen kosmetischen Mitteln handelt es sich um Entfärbemittel, welche zur Entfärbung von zuvor gefärbten keratinischen Fasern, insbesondere menschlichen Haaren eingesetzt werden. Bei den gefärbten Keratinfasern handelt es sich üblicherweise um Fasern, welche zuvor mit herkömmlichen, dem Fachmann bekannten Oxidationsfarbstoffen und/oder direktziehenden Farbstoffen gefärbt wurden.

Die Entfärbemittel sind geeignet zur Entfernung von Färbungen, welche mit Oxidationsfarbstoffen auf Basis von Entwickler- und Kupplerkomponenten auf den Keratinfasern erzeugt wurden. Wenn als Entwickler die folgenden Verbindungen einsetzt wurden, lassen sich die hiermit erzeugten Färbungen durch Einsatz des Entfärbemittel gut, effektiv und nahezu ohne spätere Nachdunklung entfernen: p-Phenylendiamin, p-ToluylendiaminN,N-Bis-(β-hydroxyethyl)-p-phenylendiamin, 4-N,N-Bis-(β-hydroxyethyl)-amino-2-methylanilin, 2-(β-Hydroxyethyl)-p-phenylendiamin, 2-(α,β-Dihydroxyethyl)-p-phenylendiamin, 2-Hydroxymethyl-p-phenylendiamin, Bis-(2-hydroxy-5-aminophenyl)-methan, p-Aminophenol, 4-Amino-3-methylphenol, 2,4,5,6-Tetraaminopyrimidin, 4-Hydroxy-2,5,6-triaminopyrimidin, 2-Hydroxy-4,5,6-triaminopyrimidin und/oder 4,5-Diamino-1-(β-hydroxyethyl)-pyrazol.

Wenn als Kuppler die folgenden Verbindungen einsetzt wurden, lassen sich die hiermit erzeugten Färbungen ebenfalls mit sehr gutem Entfärbeergebnis entfernen: m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol, 1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Entfärbemittel sind zur Entfernung dieser Färbungen gedacht und enthalten daher bevorzugt keine Farbstoffe, insbesondere keine Oxidationsfarbstoffvorprodukte vom Entwicklertyp und/oder Kupplertyp sowie direktziehenden Farbstoffe.

Bevorzugt beträgt daher die Gesamtmenge aller im dem kosmetischen Mittel enthaltenen direktziehenden Farbstoffe und/oder Oxidationsfarbstoffvorprodukte höchstens 0,2 Gew.-%, vorzugsweise höchstens 0,1 Gew.-%, bevorzugt höchstens 0,05 Gew.-%, insbesondere höchstens 0,01 Gew.-%, bezogen auf das Gesamtgewicht des kosmetischen Mittels.

Die erfindungsgemäßen kosmetischen Mittel enthalten einen kosmetisch verträglichen Träger. Erfindungsgemäß bevorzugt ist der kosmetisch verträgliche Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden. Besonders bevorzugt liegen die kosmetischen Mittel als Emulsionen vor, da bei Einsatz von Emulsionen überraschenderweise die Geruchsbelastung während der reduktiven Entfärbung verringert wird.

Ein wässriger Träger enthält im Sinne der Erfindung mindestens 30 Gew.-%, insbesondere mindestens 50 Gew.-%, Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen kosmetischen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) ein Sulfinsäurederivat der Formel (I).

Sulfinsäurederivate der Formel (I) weisen als Rest A eine Gruppierung N(R³)_{3-q} auf. Wenn A für die Gruppierung N(R³)_{3-q} steht, dann weisen die Sulfinsäurederivate die Formel N(R³)_{3-q} [(CR¹R²)SO₂M]_{q} auf.

Die besten Entfärbeleistungen konnten durch Einsatz einer Verbindung der Formel (I) erhalten werden, in welcher der Rest A für N(R³)_{3-q} steht. Das erfindungsgemäße kosmetische Mittel enthält daher ein Sulfinsäurederivat der Formel N(R³)_{3-q} [(CR¹R²)SO₂M]_{q}. In diesem Zusammenhang steht in der Formel (I) q für die Zahl 3. Bei im Rahmen der vorliegenden Erfindung eingesetzten Sulfinsäurederivaten der Formel (I) handelt es sich daher um die Verbindung der Formel N[(CR¹R²)SO₂M]₃(p = 3). Die Reste R¹ und R² in der Formel (I) stehen unabhängig voneinander für ein Wasserstoffatom. Im Hinblick auf die Entfärbeleistung der erfindungsgemäßen kosmetischen Mittel ist es bevorzugt, wenn in der Formel (I) die Reste R¹ und R², jeweils unabhängig voneinander, für ein Wasserstoffatom stehen. Es werden daher Sulfinsäurederivate der Formel N[(CH₂)SO₂M]₃ in den erfindungsgemäßen kosmetischen Mitteln eingesetzt.

In der Formel (I) steht M für identische Reste, für Natrium. Bei den erfindungsgemäßen Sulfinsäurederivaten der Formel (I) handelt es sich um Verbindungen, welche jeweils - gebunden an ein Stickstoffatom - mit den Resten R¹ und R² substituierte Sulfinomethylgruppe, die alternativ auch als Methansulfinsäure-Gruppen bezeichnet werden können, enthalten. Die Sulfinomethylgruppe liegt in Form ihres Natriumsalzes vor. Im Hinblick auf die Entfärbeergebnisse ist es vorteilhaft, wenn die erfindungsgemäßen kosmetischen Mittel ganz bestimmte Sulfinsäurederivate der Formel (I) enthalten:
- N(CH2SO2Na)3, Trinatrium [bis(sulfinatomethyl)amino]methansulfinat

Der Einsatz der zuvor angeführten Verbindungen der Formel (la), welche auch als Trinatrium[bis(sulfinatomethyl)amino]methansulfinat bezeichnet werden, in den erfindungsgemäßen kosmetischen Mitteln führt zu einer hervorragenden Entfärbeleistung, insbesondere auf mit oxidativen Färbemitteln gefärbten keratinischen Fasern. Nach der Entfärbung tritt zudem keinerlei Rückfärbung oder Nachdunklung auf.

Das bzw. die Sulfinsäurederivat der Formel (I) wird bzw. werden bevorzugt in bestimmten Mengenbereichen eingesetzt. Es ist daher erfindungsgemäß vorteilhaft, wenn die mindestens eine Verbindung I der Formel (I) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-%, bezogen auf das Gesamtgewicht des kosmetischen Mittels, enthalten ist. Unter Gesamtmenge wird hierbei die Menge aller im erfindungsgemäßen kosmetischen Mittel enthaltenden Verbindungen I der Formel (I) verstanden.

Als zweiten wesentlichen Bestandteil b) enthält das erfindungsgemäße kosmetische Mittel mindestens einen Alkohol mit 10 bis 30 Kohlenstoffatomen in einer Gesamtmenge von 0,1 bis 20 Gew.-%, bezogen auf das Gesamtgewicht des kosmetischen Mittels.

Besonders bevorzugt ist der mindestens eine Alkohol b) mit 10 bis 30 Kohlenstoffatomen ausgewählt aus der Gruppe von 1-Decanol, Laurylalkohol, Myristylalkohol, Cetylalkohol, Stearylalkohol, Arachidylalkohol, Behenylalkohol, Lignocerylalkohol, Cerylalkohol, Montanylalkohol sowie deren Mischungen, insbesondere aus einer Mischung von Cetylalkohol und Stearylalkohol. Der Einsatz der zuvor angeführten Alkohole in den erfindungsgemäßen kosmetischen Mitteln führt zu einer verminderten Geruchsbelastung während der reduktiven Entfärbung von gefärbten keratinischen Fasern mit den erfindungsgemäßen kosmetischen Mitteln.

Es ist im Rahmen der vorliegenden Erfindung vorteilhaft, wenn der mindestens eine Alkohol b) in einer bestimmten Gesamtmenge eingesetzt wird. Bevorzugte erfindungsgemäße kosmetische Mittel enthalten daher den mindestens einen Alkohol b) in einer Gesamtmenge von 0,5 bis 18 Gew.-%, vorzugsweise von 1,0 bis 15 Gew.-%, bevorzugt von 1,2 bis 12 Gew.-%, insbesondere von 1,5 bis 10 Gew.-%, bezogen auf das Gesamtgewicht des kosmetischen Mittels.

Zusätzlich zu der Verbindung der Formel (I) kann in dem erfindungsgemäßen kosmetischen Mittel eine Sulfonsäure der Formel (II) eingesetzt werden. Bevorzugt enthält das kosmetische Mittel daher zusätzlich eine Verbindung II der Formel (II)

A[(CR¹R²)SO₃M]_{p,q} (II)

in welcher
- A: für N(R³)_{3-q} oder O(R⁴)₂₋ₚ steht
- R¹, R², R⁴: unabhängig voneinander für ein Wasserstoffatom oder für eine C₁-C₆-Alkylgruppe stehen,
- R³: für identische oder verschiedene Reste ausgewählt aus einem Wasserstoffatom, einer C₁-C₂₀-Alkylgruppe, einer C₃-C₈-Cycloalkylgruppe ggf. substituiert durch ein bis drei C₁-C₄-Alkylreste steht
- M: für identische oder verschiedene Reste ausgewählt aus einem Wasserstoffatom oder einem Äquivalent eines Alkali-, Erdalkali- oder Metallions, insbesondere für Natrium, Kalium, ½ Magnesium, ½ Calcium, ½ Zink, oder für ein Ammoniumion (NH₄⁺) steht,
- p: für die Zahlen 1 oder 2 steht,
- q: für die Zahlen 1, 2 oder 3 steht,
wobei
- mindestens einer der Reste R¹, R², R⁴ für eine C₁-C₆-Alkylgruppe steht, wenn A für O(R⁴₂₋ₚ) steht, und
- R³ nicht für ein Wasserstoffatom steht, wenn q gleich 1 ist, dadurch gekennzeichnet, dass es mindestens eine Verbindung II der Formel (IIa) N(CH₂SO₃Na)₃ enthält.

Bezüglich der Reste A, R¹ bis R⁴ und M sowie p und q der Formel (II) wird auf obige Ausführungen verwiesen, welche für diese Ausführungsform gleichermaßen gelten.

Sulfonsäurederivate der Formel (II) weisen als Rest A eine Gruppierung N(R³)_{3-q} auf. Wenn A für die Gruppierung N(R³)_{3-q} steht, dann weisen die Sulfonsäurederivate die Formel N(R³)_{3-q} [(CR¹R²)SO₃M]_{q} auf.

Eine verbesserte Entfärbeleistung konnte durch den zusätzlichen Einsatz einer Verbindung der Formel (II) erhalten werden, in welcher der Rest A für N(R³)_{3-q} steht. Das erfindungsgemäße kosmetische Mittel enthält daher ein Sulfonsäurederivat der Formel N(R³)_{3-q}[(CR¹R²)SO₃M]_{q}. In diesem Zusammenhang steht in der Formel (II) q für die Zahl 3. Bei im Rahmen der vorliegenden Erfindung eingesetzten Sulfonsäurederivaten der Formel (II) handelt es sich daher um die Verbindungen der Formel N[(CR¹R²)SO₃M]₃(p = 3). Die Reste R¹ und R² in der Formel (II) stehen unabhängig voneinander für ein Wasserstoffatom Im Hinblick auf die Entfärbeleistung der erfindungsgemäßen kosmetischen Mittel ist es bevorzugt, wenn in der Formel (II) die Reste R¹ und R²,jeweils unabhängig voneinander, für ein Wasserstoffatom stehen. Es werden daher Sulfonsäurederivate der Formeln N[(CH₂)SO₃M]₃ in den erfindungsgemäßen kosmetischen Mitteln eingesetzt. In der Formel (II) steht M für identische Reste, für Natrium. Bei den Sulfonsäurederivaten der Formel (II) handelt es sich um Verbindungen, welche jeweils - gebunden an ein Stickstoffatom - mit den Resten R¹ und R² substituierte Sulfomethylgruppe, die alternativ auch als Methansulfonsäure-Gruppen bezeichnet werden können, enthalten. Die Sulfomethylgruppe liegt in Form ihres Natriumsalzes vor.

Es ist erfindungsgemäß, wenn in der Formel (II) M für identische Reste, für Natrium steht. Im Hinblick auf die Entfärbeergebnisse ist es vorteilhaft, wenn die erfindungsgemäßen kosmetischen Mittel zusätzlich ein ganz bestimmtes Sulfonderivat der Formel (II) enthalten: - N(CH₂SO₃Na)₃, Trinatrium [bis(sulfonatomethyl)amino]methansulfonat

Der Einsatz der zuvor angeführten Verbindungen der Formel (IIa), welche auch als Trinatrium[bis(sulfinatomethyl)amino]methansulfonat bezeichnet werden, in Kombination mit Verbindungen I der Formel (I) bzw. (la) führt zu einer hervorragenden Entfärbeleistung, insbesondere auf mit oxidativen Färbemitteln gefärbten keratinischen Fasern. Nach der Entfärbung tritt zudem keinerlei Rückfärbung oder Nachdunklung auf.

Das bzw. die Sulfonsäurederivat(e) der Formel (II) wird bzw. werden bevorzugt in bestimmten Mengenbereichen eingesetzt. Es ist daher erfindungsgemäß vorteilhaft, wenn die mindestens eine Verbindung II der Formel (II) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-%, bezogen auf das Gesamtgewicht des kosmetischen Mittels, enthalten ist. Unter Gesamtmenge wird hierbei die Menge aller im erfindungsgemäßen kosmetischen Mittel enthaltenden Verbindungen II der Formel (II) verstanden.

Es sind daher kosmetische Mittel zur reduktiven Entfärbung gefärbter keratinischer Fasern vorteilhaft, welche in einem kosmetischen Träger
a) mindestens eine Verbindung I der Formel (I)

   A[(CR¹R²)SO₂M]_{p,q} (I)

   in welcher
   - A: für N(R³)_{3-q} oder O(R⁴)₂₋ₚ steht
   - R¹, R², R⁴: unabhängig voneinander für ein Wasserstoffatom oder für eine C₁-C₆-Alkylgruppe stehen,
   - R³: für identische oder verschiedene Reste ausgewählt aus einem Wasserstoffatom, einer C₁-C₂₀-Alkylgruppe, einer C₃-C₈-Cycloalkylgruppe ggf. substituiert durch ein bis drei C₁-C₄-Alkylreste steht
   - M: für identische oder verschiedene Reste ausgewählt aus einem Wasserstoffatom oder einem Äquivalent eines Alkali-, Erdalkali- oder Metallions, insbesondere für Natrium, Kalium, ½ Magnesium, ½ Calcium, ½ Zink, oder für ein Ammoniumion (NH₄⁺) steht,
   - p: für die Zahlen 1 oder 2 steht,
   - q: für die Zahlen 1, 2 oder 3 steht,
   wobei
   - mindestens einer der Reste R¹, R², R⁴ für eine C₁-C₆-Alkylgruppe steht, wenn A für O(R⁴₂₋ₚ) steht, und
   - R³ nicht für ein Wasserstoffatom steht, wenn q gleich 1 ist,
b) mindestens einen Alkohol mit 10 bis 30 Kohlenstoffatomen in einer Gesamtmenge von 0,1 bis 20 Gew.-%, bezogen auf das Gesamtgewicht des kosmetischen Mittels, und
c) mindestens eine Verbindung II der Formel (II)

   A[(CR¹R²)SO₃M]_{p,q} (II)

   in welcher A, R¹, R², R³, R⁴, M, p und q dieselbe allgemeine Bedeutung wie in Formel (I) besitzen, wobei die Auswahl dieser Variablen im konkreten Einzelfall für die Verbindungen der Formeln (I) und (II) nicht gleich sein muss, enthalten, welche
   a) mindestens eine Verbindung I der Formel (la)

      N(CH₂SO₂Na)₃ (la),

      und
   b) mindestens eine Verbindung II der Formel (IIa)

      N(CH₂SO₃Na)₃ (IIa),

      umfassen.

Enthalten die erfindungsgemäßen reduktiven Entfärbemittel sowohl mindestens eine Verbindung I der Formel (I) bzw. (la) als auch mindestens eine Verbindung II der Formel (II) bzw. (IIa) werden sie bevorzugt in bestimmten molaren Mengenverhältnissen eingesetzt. Es sind daher kosmetische Mittel bevorzugt, welche ein molares Verhältnis der Verbindungen I der Formel (I) bzw. (la) zu den Verbindungen II der Formel (II) bzw. (IIa) von 20:1 bis 1:20, vorzugsweise von 10:1 bis 1:10, insbesondere von 3:1 bis 1:3, aufweisen.

Gemäß einer besonders bevorzugten Ausführungsform der vorliegenden Erfindung liegen die kosmetischen Mittel als Emulsion vor. Die Verwendung der zuvor angeführten Verbindungen der Formeln (I) bzw. (la) sowie gegebenenfalls Verbindungen der Formeln (II) bzw. (IIa) in Kombination mit dem speziellen Alkohol b) als Emulsion führt im Vergleich zu der Verwendung der zuvor genannten Verbindungen als Gel oder als wässrige Lösung zu einer signifikant verminderten Geruchsbelastung während der reduktiven Entfärbung gefärbter keratinischer Fasern. Zusätzlich kann das erfindungsgemäße kosmetische Mittel neben der Verbindung I der Formel (I bzw. (Ia) und gegebenenfalls der Verbindung (II) der Formel (II) bzw. (IIa) mindestens ein weiteres Reduktionsmittel aus der Gruppe von Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Natriumdisulfit, Kaliumdisulfit, Ammoniumdisulfit, Hydroxymethansulfinsäure, Aminomethansulfinsäure, Cystein, Thiomilchsäure, Thioglycolsäure (Alternativname: Sulfanylessigsäure), Oxalsäure, Oxalessigsäure und/oder Ascorbinsäure enthalten. Unter Reduktionsmitteln werden hierbei chemische Verbindungen verstanden, welche in der Lage sind, andere chemische Verbindungen unter Abgabe von Elektronen zu reduzieren.

Bei Natriumdithionit handelt es sich um ein anorganischen Reduktionsmittel mit der Summenformel Na₂S₂O₄ und der CAS-Nr. 7775-14-6. Bei Zinkdithionit handelt es sich um ein anorganisches Reduktionsmittel mit der Summenformel ZnS₂O₄ und der CAS-Nr. 7779-86-4. Bei Kaliumdithionit handelt es sich um ein anorganisches Reduktionsmittel mit der Summenformel K₂S₂O₄ und der CAS-Nr. 14293-73-3. Bei Natriumsulfit handelt es sich um ein anorganisches Reduktionsmittel mit der Summenformel Na₂SO₃ und der CAS-Nr. 7757-83-7.

Bei Natriumhydrogensulfit handelt es sich um ein anorganisches Reduktionsmittel mit der Summenformel NaHSO₃ und der CAS-Nr. 7631-90-5. Natriumhydrogensulfit wird bevorzugt in Form einer wässrigen Lösung eingesetzt.

Bei Kaliumsulfit handelt es sich um ein anorganisches Reduktionsmittel mit der Summenformel K₂SO₃ und der CAS-Nr. 10117-38-1. Bei Kaliumhydrogensulfit handelt es sich um ein anorganisches Reduktionsmittel mit der Summenformel KHSO₃ und der CAS-Nr. 7773-03-7. Bei Ammoniumsulfit handelt es sich um ein anorganisches Reduktionsmittel mit der Summenformel (NH₄)₂SO₃ und der CAS-Nr. 10196-04-0. Bei Natriumthiosulfat handelt es sich um ein anorganisches Reduktionsmittel mit der Summenformel Na₂S₂O₃ und der CAS-Nr. 7772-98-7. Bei Kaliumthiosulfat handelt es sich um ein anorganisches Reduktionsmittel mit der Summenformel K₂S₂O₃ und der CAS-Nr. 10294-66-3. Bei Ammoniumthiosulfat handelt es sich um ein anorganisches Reduktionsmittel mit der Summenformel (NH₄)₂S₂O₃ und der CAS-Nr. 7783-18-8.

Bei Hydroxymethansulfinsäure handelt es sich um ein organisches Reduktionsmittel mit der Formel HO-CH₂-S(O)OH und der CAS-Nr. 79-25-4. Alternativ wird Hydroxymethansulfinsäure auch als Formaldehydsulfoxylsäure bezeichnet. Erfindungsgemäß kann sowohl Hydroxymethansulfinsäure als auch deren physiologisch verträglichen Salze, beispielsweise Natriumsalze und/oder Zinksalze, eingesetzt werden.

Bei Aminomethansulfinsäure handelt es sich um ein organisches Reduktionsmittel der Formel H₂N-CH₂-S(O)OH und der CAS-Nr. 118201-33-5. Erfindungsgemäß ist sowohl der Einsatz der Aminomethansulfinsäure selbst als auch der Einsatz der physiologisch verträglichen Salze von Aminomethansulfinsäure, beispielsweise des Natriumsalzes und/oder des Zinksalzes. Der Einsatz von Natrium Aminomethansulfinat (dem Natriumsalz der Aminomethansulfinsäure) und/oder Zink Aminomethansulfinat (dem Zinksalz der Aminomethansulfinsäure) ist deshalb ebenfalls erfindungsgemäß.

Unter Cystein (2-Amino-3-sulfanylpropionsäure) wird erfindungsgemäß D-Cystein, L-Cystein und/oder ein Gemisch aus D- und L-Cystein verstanden.

Unter Thiomilchsäure (2-Sulfanylpropionsäure) wird D-Thio-Milchsäure, L-Thio-Milchsäure und/oder ein Gemisch aus D- und L-Thiomilchsäure verstanden. Erfindungsgemäß ist sowohl der Einsatz der Thiomilchsäure selbst als auch der Einsatz von Thiomilchsäure in Form eines physiologisch verträglichen Salzes hiervon. Ein bevorzugtes Salz der Thiomilchsäure ist Ammoniumthiolactat.

Bei Ammoniumthiolactat handelt es sich um das Ammoniumsalz der Thiomilchsäure (d.h. das Ammoniumsalz der 2-Sulfanylpropionsäure) mit der Formel (III) 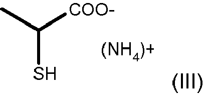 wobei unter Ammoniumthiolactat erfindungsgemäß die Ammoniumsalze der D-Thiomilchsäure, die Ammoniumsalze der L-Thiomilchsäure sowie deren Gemische verstanden werden.

Unter Thioglycolsäure (Sulfanylessigsäure, 2-Mercapto-essigäure) wird ein organisches Reduktionsmittel der Formel HS-CH₂-COOH und der CAS-Nr. 68-11-1 verstanden. Auch bei Thioglycolsäure ist sowohl der Einsatz von Thioglycolsäure selbst auch der Einsatz eines physiologisch verträglichen Salzes der Thioglycolsäure erfindungsgemäß. Als physiologisch verträgliche Salze der Thiolgycolsäure können beispielsweis Natriumthioglycolat, Kaliumthioglycolat und/oder Ammoniumthioglycolat eingesetzt werden. Ammoniumthioglylat ist ein bevorzugtes physiologisch verträgliches Salze der Thioglycolsäure.

Bei Ammoniumthioglycolat handelt es sich um das Ammoniumsalz der Thiglycolsäure (d.h. das Ammoniumsalz der Sulfanylessigsäure) mit der Formel (IV)

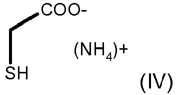

Unter Oxalsäure wird die reduzierend wirkende Säure HOOC-COOH verstanden. Oxalsäure wird alternativ auch als Ethandisäure bezeichnet und trägt die CAS-Nr. 144-62-7. Alternativnamen für Oxalessigsäure sind auch Oxobutandisäure oder Oxobernsteinsäure, diese Säure wirkt ebenfalls reduzierend und trägt die CAS-Nr. 328-42-7. Unter Ascorbinsäure wird erfindungsgemäß insbesondere (*R*)-5-[(*S*)-1,2-Dihydroxyethyl]-3,4-dihydroxy-5*H*-furan-2-on (weitere Alternativnamen: Vitamin C, L-Ascorbinsäure) mit der CAS-Nr. 50-81-7 verstanden.

Das mindestens eine zusätzliche Reduktionsmittel aus der vorgenannten Gruppe kann in einer Gesamtmenge von 0,1 bis 20,0 Gew.-%, vorzugsweise von 0,5 bis 10,0 Gew.-%, insbesondere von 1,0 bis 8,0 Gew.-%, bezogen auf das Gesamtgewicht des kosmetischen Mittels, enthalten sein. Die erfindungsgemäßen kosmetischen Mittel können weiterhin zusätzlich mindestens ein Tensid aus der Gruppe der anionischen Tenside, der amphoteren und/oder zwitterionischen Tenside, der nichtionischen Tenside, der kationischen Tenside sowie deren Mischungen enthalten. Der zusätzliche Einsatz von Tensiden führt zu einer Stabilisierung der Emulsion und unterstützt damit die verminderte Geruchsbelastung bei Anwendung der erfindungsgemäßen kosmetischen Mittel während der reduktiven Entfärbung gefärbter keratinischer Fasern.

Tenside sind amphiphile (bifunktionelle) Verbindungen, welche aus mindestens einem hydrophoben und mindestens einem hydrophilen Molekülteil bestehen. Bei dem hydrophoben Rest handelt es sich bevorzugt um eine Kohlenwasserstoffkette mit 8 bis 24 Kohlenstoffatomen, welche gesättigt oder ungesättigt, linear oder verzweigt sein kann. Besonders bevorzugt ist diese C₈-C₂₄-Alkylkette linear. Anionische Tenside im Sinne der vorliegenden Erfindung enthalten mindestens eine anionische Gruppe, beispielsweise eine Carboxylat-, Sulfat-, Sulfonat- oder Phosphat-Gruppe, sowie eine lipophile Alkylgruppe mit 8 bis 30 Kohlenstoffatomen. Zusätzlich können im Molekül Glykol- oder Polyglykolether-Gruppen, Ester-, Ether- und Amidgruppen sowie Hydroxylgruppen enthalten sein. Ein anionisches Tensid im Sinne der vorliegenden Erfindung enthält jedoch keine kationischen Gruppierungen, d.h. zwitterionische Tenside fallen daher nicht unter die erfindungsgemäße Definition anionischer Tenside.

Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Beispiele für erfindungsgemäße anionische Tenside sind, jeweils in Form der Natrium-, Kalium- und Ammonium- sowie der Mono-, Di- und Trialkanolammoniumsalze mit 2 bis 4 Kohlenstoffatomen in der Alkanolgruppe,
- lineare und verzweigte Fettsäuren mit 8 bis 30 Kohlenstoffatomen (Seifen),
- Ethercarbonsäuren der Formel R⁵-O-(CH₂-CH₂O)ₓ-CH₂-COOH, in welcher R⁵ eine lineare Alkylgruppe mit 8 bis 30 Kohlenstoffatomen und x = 0 oder 1 bis 16 ist,
- Acylsarcoside mit 8 bis 24 Kohlenstoffatomen in der Acylgruppe,
- Acyltauride mit 8 bis 24 Kohlenstoffatomen in der Acylgruppe,
- Acylisethionate mit 8 bis 24, insbesondere 12 bis 18, Kohlenstoffatomen in der Acylgruppe, welche durch Veresterung von Fettsäuren mit dem Natriumsalz der 2-Hydroxyethan-sulfonsäure (Isethionsäure) zugänglich sind,
- Sulfobernsteinsäuremono- und -dialkylester mit 8 bis 24, insbesondere 12 bis 18, Kohlenstoffatomen in der Alkylgruppe und Sulfobernsteinsäuremonoalkylpolyoxyethylester mit 8 bis 24, insbesondere 12 bis 18, Kohlenstoffatomen in der Alkylgruppe und 1 bis 6 Oxyethylgruppen,
- lineare Alkansulfonate mit 8 bis 24 Kohlenstoffatomen,
- lineare Alpha-Olefinsulfonate mit 8 bis 24 Kohlenstoffatomen,
- Alpha-Sulfofettsäuremethylester von Fettsäuren mit 8 bis 30 Kohlenstoffatomen,
- Alkylsulfate und Alkylpolyglykolethersulfate der Formel R⁶-O(CH₂-CH₂O)ₓ-OSO₃H, in welcher R⁶ eine bevorzugt lineare Alkylgruppe mit 8 bis 30 Kohlenstoffatomen und x = 0 oder 1 bis 12 ist,
- Hydroxysulfonate im wesentlichen entsprechend mindestens einer der beiden folgenden Formeln oder deren Mischungen sowie deren Salzen, CH₃-(CH₂)_{y}-CHOH-(CH₂)ₐ-(CH-SO₃M)-(CH₂)_{z}-CH₂-O-(CₙH₂ₙO)ₓ-H, und/oder CH₃-(CH₂)_{y}-(CH-SO₃M)-(CH₂)ₐ-CHOH-(CH₂)_{z}-CH₂-O-(CₙH₂ₙO)ₓ-H wobei in beiden Formeln y und z = 0 oder ganze Zahlen von 1 bis 18, a = 0, 1 oder 2 und die Summe (y+z+a) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C₁ bis C₄-Alkyl-, Alkenyl- oder Arylresten,
- sulfatierte Hydroxyalkylpolyethylen- und/oder Hydroxyalkylenpropylenglykolether der Formel R⁷-(CHOSO₃M)-CHR⁹-(OCHR¹⁰-CH₂)ₙ-OR⁸ mit R⁷, einem linearen Alkylrest mit 1 bis 24 Kohlenstoffatomen, R⁸ für einen linearen oder verzweigten, gesättigten Alkylrest mit 1 bis 24 Kohlenstoffatomen, R⁹ für Wasserstoff oder einen linearen Alkylrest mit 1 bis 24 Kohlenstoffatomen, R¹⁰ für Wasserstoff oder einen Methylrest und M für Wasserstoff, Ammonium, Alkylammonium, Alkanolammonium, worin die Alkyl- und Alkanolreste je 1 bis 4 Kohlenstoffatome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in R¹ und R³ enthaltenen Kohlenstoffatomen 2 bis 44 beträgt,
- Sulfonate ungesättigter Fettsäuren mit 8 bis 24 Kohlenstoffatomen und 1 bis 6 Doppelbindungen,
- Ester der Weinsäure und Zitronensäure mit Alkoholen, welche Anlagerungsprodukte von etwa 2 bis 15 Molekülen Ethylenoxid und/oder Propylenoxid an Fettalkohole mit 8 bis 22 Kohlenstoffatomen darstellen,
- Alkyl- und/oder Alkenyletherphosphate der Formel R¹¹(OCH₂CH₂)ₙ-O-(PO-OX)-OR¹², in welcher R¹¹ bevorzugt für einen aliphatischen Kohlenwasserstoffrest mit 8 bis 30 Kohlenstoffatomen, R¹² für Wasserstoff, einen Rest (CH₂CH₂O)ₙR¹² oder X, n für Zahlen von 1 bis 10 und X für Wasserstoff, ein Alkali- oder Erdalkalimetall oder NR¹³R¹⁴R¹⁵R¹⁶, mit R¹³ bis R¹⁶ unabhängig voneinander stehend für Wasserstoff oder einen C₁-C₄-Kohlenwasserstoffrest, steht,
- sulfatierte Fettsäurealkylenglykolester der Formel R¹⁷CO(AlkO)ₙSO₃M, in welcher R¹⁷CO- für einen linearen oder verzweigten, aliphatischen, gesättigten und/oder ungesättigten Acylrest mit 6 bis 22 Kohlenstoffatomen, Alk für CH₂CH₂, CHCH₃CH₂ und/oder CH₂CHCH₃, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie ⁺ NR¹³R¹⁴R¹⁵R¹⁶, mit R¹³ bis R¹⁶ unabhängig voneinander stehend für Wasserstoff oder einen C₁-C₄-Kohlenwasserstoffrest,
- Monoglyceridsulfate und Monoglyceridethersulfate der Formel R¹⁸OC-(OCH₂CH₂)ₓ-OCH₂-[CHO(CH₂CH₂O)_{y}H]-CH₂O(CH₂CH₂O)_{z}-SO₃X, in welcher R¹⁸CO für einen linearen oder verzweigten Acylrest mit 6 bis 22 Kohlenstoffatomen, insbesondere für einen linearen Acylrest mit 8 bis 18 Kohlenstoffatomen, x, y und z in Summe für 0 oder für Zahlen von 1 bis 30, vorzugsweise 2 bis 10, und X für ein Alkali- oder Erdalkalimetall steht,
- Amidethercarbonsäuren der Formel R¹⁹-CO-NR²⁰-CH₂CH₂-O-(CH₂CH₂O)ₙCH₂COOM, mit R¹⁹ als geradkettigen oder verzweigten Alkyl- oder Alkenylrest mit einer Zahl an Kohlenstoffatomen in der Kette von 2 bis 30, n steht für eine ganze Zahl von 1 bis 20 und R²⁰ steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺ NR¹³R¹⁴R¹⁵R¹⁶, mit R¹³ bis R¹⁶ unabhängig voneinander stehend für Wasserstoff oder einen C₁-C₄-Kohlenwasserstoffrest. Derartige Produkte sind beispielsweise von der Firma Chem-Y unter der Produktbezeichnung Akypo® erhältlich, und
- Acylglutamate der Formel XOOC-CH₂CH₂CH(C(NH)OR²¹)-COOX, in welcher R²¹CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

Das erfindungsgemäße kosmetische Mittel kann ebenfalls mindestens ein amphoteres und/oder zwitterionisches Tensid enthalten. Im Fall der zwitterionischen Tenside umfasst der hydrophile Molekülteil eine zwitterionische Struktureinheit, d.h. eine Struktureinheit, welche sowohl einen kationisch geladenen als auch einen anionisch geladenen Molekülteil umfasst. Erfindungsgemäß besonders geeignete zwitterionische Tenside sind dadurch gekennzeichnet, dass sie einen kationisch geladenen Molekülteil in Form einer quartären Ammoniumgruppe besitzen und ihr anionischer Molekülteil in Form einer Gruppierung -SO₃⁻ oder -COO⁻ vorliegt. Eine Ammoniumgruppe ist quartär, wenn eine Gruppierung des Typs (RₐR_{b}R_{c}R_{d}N)⁺ vorliegt, d.h. wenn alle vier H-Atome des NH₄-Ions, von dem die quartäre Ammoniumgruppe abgeleitet ist, durch organische Reste R (bzw. Rₐ bis R_{d}) ersetzt wird. Die Gruppierung -SO₃⁻ des zwitterionischen Tensids kann direkt an ein Kohlenstoffatom gebunden vorliegen. In diesem Fall handelt es sich bei dem anionischen Teil der zwitterionischen Verbindung um eine deprotonierte Sulfonsäuregruppierung.

Besonders geeignete zwitterionische Tenside sind beispielsweise Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline, N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, das Kokosacylaminoethylaminopropionat und C₁₂-C₁₈-Acylsarcosin.

Weiterhin hat es sich als vorteilhaft erwiesen, wenn das Mittel weitere, nichtionogene grenzflächenaktive Stoffe, enthält. Bevorzugte nichtionische Tenside sind Alkylpolyglykoside sowie Alkylenoxid-Anlagerungsprodukte an Fettalkohole, Fettsäuren und Fettsäureglyceride mit jeweils 2 bis 50 Mol Ethylenoxid pro Mol Fettalkohol bzw. Fettsäure. Als nichtionische Tenside sind außerdem Fettsäureester von ethoxyliertem Glycerin vorteilhaft. Ganz besonders bevorzugt ist es, wenn das kosmetische Mittel als nichtionisches Tensid ein ethoxyliertes Rizinusöl mit jeweils 2 bis 50 Mol Ethylenoxyid pro Mol Fettsäure oder ein ethoxyliertes, hydriertes Rizinusöl mit jeweils 2 bis 50 Mol Ethylenoxyid pro Mol Fettsäure enthält. Der Einsatz von PEG-40 Castor Oil ist in diesem Zusammenhang besonders bevorzugt.

Die erfindungsgemäßen Mittel können auch mindestens ein kationisches Tensid enthalten. Unter kationischen Tensiden werden Tenside verstanden, welche ausschließlich temporär oder permanent positive Gruppe enthalten. Temporär kationische Gruppe sind solche, welche in Abhängigkeit vom pH-Wert des erfindungsgemäßen kosmetischen Mittels eine kationische Gruppe aufweisen. Als permanent kationische Gruppen werden solche Gruppen bezeichnet, welche unabhängig vom pH-Wert des erfindungsgemäßen kosmetischen Mittels kationisch sind. Dies sind beispielsweise Gruppen, welche ein quartäres Stickstoffatom enthalten.

Beispiele für kationische Tenside sind
- quartäre Ammoniumverbindungen, welche als hydrophobe Reste ein oder zwei Alkylketten mit einer Kettenlänge von 8 bis 28 Kohlenstoffatomen tragen können
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das kationische Tensid auch weitere ungeladene funktionelle Gruppen beinhalten, dies ist beispielsweise bei Esterquats der Fall.

Erfindungsgemäß werden als kationische Tenside insbesondere solche vom Typ der quartären Ammoniumverbindungen, der Esterquats und der Amidoamine eingesetzt. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bei Esterquats handelt es sich um bekannte Stoffe, welche sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Weitere erfindungsgemäß verwendbare kationische Tenside stellen die quaternisierten Proteinhydrolysate dar.

Die anionischen, amphoteren bzw. zwitterionischen, nichtionischen und kationischen Tenside können jeweils in einer Gesamtmenge von 0,01 bis 15 Gew.-%, vorzugsweise von 0,1 bis 10 Gew.-%, bevorzugt von 0,2 bis 5,0 Gew.-%, insbesondere von 0,3 bis 2,0 Gew.-%, bezogen auf das Gesamtgewicht des kosmetischen Mittels, enthalten sein.

Weiterhin können die erfindungsgemäßen kosmetischen Mittel zusätzlich mindestens ein Polyol enthalten. Unter einem Polyol wird eine Verbindung mit mindestens zwei aliphatischen (d.h. nicht phenolischen) OH-Gruppen verstanden.

Bevorzugte kosmetische Mittel sind daher dadurch gekennzeichnet, dass sie zusätzlich mindestens ein Polyol aus der Gruppe von Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol, Polypropylenglycol sowie deren Mischungen enthalten.

Die Polyole werden in den erfindungsgemäßen Mitteln bevorzugt in einer Gesamtmenge von 0,01 bis 5,0 Gew.-%, vorzugsweise von 0,1 bis 4,0 Gew.-%, bevorzugt von 0,2 bis 3,0 Gew.-%, insbesondere von 0,3 bis 2,5 Gew.-%, bezogen auf das Gesamtgewicht des kosmetischen Mittels, eingesetzt.

Bevorzugt werden die erfindungsgemäßen kosmetischen Mittel als fließfähige Zubereitungen formuliert. Dabei sollten die kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es ist daher im Rahmen der vorliegenden Erfindung bevorzugt, wenn die erfindungsgemäßen kosmetischen Mittel zusätzlich mindestens ein Verdickungsmittel, ausgewählt aus der Gruppe von anionischen synthetischen Polymeren, kationischen synthetischen Polymeren, nichtionischen Guargums, Skleroglucangums, Xanthangums, Gummi arabicum, Carrageen-Gummi, Agar-Agar, Johannisbrotkernmehl, Pektinen, Alginaten, Stärken, Cellulosen und Cellulosederivaten, nichtionischen synthetischen Polymeren sowie deren Mischungen, enthalten.

Es hat sich in diesem Zusammenhang als vorteilhaft erwiesen, wenn das Verdickungsmittel in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,3 Gew.-%, bezogen auf das Gesamtgewicht des kosmetischen Mittels, enthalten ist.

Die erfindungsgemäßen kosmetischen Mittel weisen bevorzugt eine gewisse Viskosität auf, um einerseits eine gute Verteilbarkeit zu gewährleisten und andererseits ein Herablaufen dieser Mittel aus den Haaren zu verhindern. Es ist daher erfindungsgemäß bevorzugt, wenn das kosmetische Mittel eine Viskosität, gemessen bei 20 °C mit einem Brookfield Rotationsviskositmeter, Spindel 5, 20 rpm, von 100 bis 100.000 mPa*s, vorzugsweise von 300 bis 80.000 mPa*s, bevorzugt von 500 bis 60.000 mPa*s, weiter bevorzugt von 700 bis 40.000 mPa*s, insbesondere von 1.000 bis 30.000 mPa*s, aufweist.

Die Entfärbewirkung der erfindungsgemäßen kosmetischen Mittel kann durch Einstellung bestimmter pH-Werte optimiert werden. Eine höhere Entfärbewirkung wurde hierbei bei sauren pH-Werten im Bereich von 0,5 bis 5,0 beobachtet. Es ist daher erfindungsgemäß vorteilhaft, wenn die kosmetischen Mittel einen pH-Wert, gemessen bei 20 °C, von pH 0,5 bis pH 5,0, vorzugsweise von pH 0,6 bis pH 4,5, insbesondere von pH 1,0 bis pH 4,0, aufweisen. Die Einstellung des pH-Wertes erfolgt durch die Verwendung von Säuren. Bevorzugt wird hierfür im Rahmen der vorliegenden Erfindung mindestens eine Säure aus der Gruppe von Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure), Oxalessigsäurediester, 1-Hydroxyethan-1,1-diphosphonsäure sowie deren Mischungen eingesetzt. In nachfolgenden Tabellen sind besonders bevorzugte Ausführungsformen (AF) der erfindungsgemäßen kosmetischen Mittel aufgeführt, wobei alle Mittel als Emulsion vorliegen und einen pH-Wert von 1,0 bis pH 4,0 (gemessen bei 20 °C) aufweisen (alle Angaben in Gew.-%, sofern nichts anderes angegeben ist):

| | AF 1 | AF 2 | AF 3 | AF4 |
|---|---|---|---|---|
| Verbindung I Formel (I) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Kosmetischer Träger | Ad 100 | Ad 100 | Ad 100 | Ad 100 |

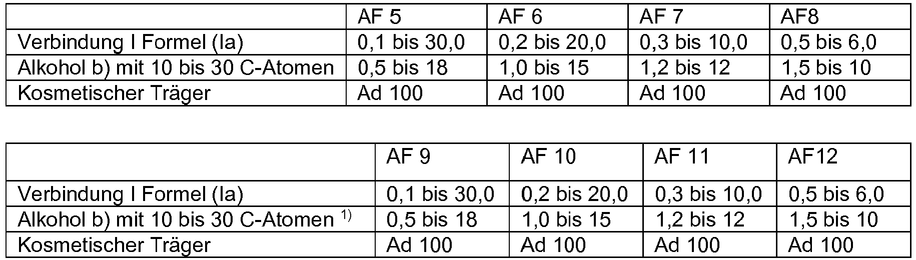

| | AF 13 | AF 14 | AF 15 | AF16 |
|---|---|---|---|---|
| Verbindung I Formel (Ia) | 0,1 bis 30,0 | 0,2 bis 20,0 | 0,3 bis 10,0 | 0,5 bis 6,0 |
| Mischung aus Cetylalkohol und Stearylalkohol | 0,5 bis 18 | 1,0 bis 15 | 1,2 bis 12 | 1,5 bis 10 |
| Kosmetischer Träger | Ad 100 | Ad 100 | Ad 100 | Ad 100 |

| | AF 17 | AF 18 | AF 19 | AF 20 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Xanthan | 0,0005 bis 5,0 | 0,001 bis 1,0 | 0,005 bis 0,5 | 0,01 bis 0,3 |
| Kosmetischer Träger | Ad 100 | Ad 100 | Ad 100 | Ad 100 |

| | AF 21 | AF 22 | AF 23 | AF 24 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Kosmetischer Träger | Ad 100 | Ad 100 | Ad 100 | Ad 100 |

| | AF 25 | AF 26 | AF 27 | AF 28 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Reduktionsmittel ³⁾ | 0,05 bis 5,0 | 0,1 bis 4,0 | 0,2 bis 3,0 | 0,3 bis 2,0 |
| Kosmetischer Träger | Ad 100 | Ad 100 | Ad 100 | Ad 100 |

| | AF 29 | AF 30 | AF 31 | AF 32 |
|---|---|---|---|---|
| Verbindung I Formel (Ia) | 0,1 bis 30,0 | 0,2 bis 20,0 | 0,3 bis 10,0 | 0,5 bis 6,0 |
| Mischung aus Cetylalkohol und Stearylalkohol | 0,5 bis 18 | 1,0 bis 15 | 1,2 bis 12 | 1,5 bis 10 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Reduktionsmittel ³⁾ | 0,05 bis 5,0 | 0,1 bis 4,0 | 0,2 bis 3,0 | 0,3 bis 2,0 |
| Kosmetischer Träger | Ad 100 | Ad 100 | Ad 100 | Ad 100 |

| | AF 33 | AF 34 | AF 35 | AF 36 |
|---|---|---|---|---|
| Verbindung I Formel (I) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Verbindung II Formel (II) | 0,1 bis 30,0 | 0,2 bis 20,0 | 0,3 bis 10,0 | 0,5 bis 6,0 |
| Kosmetischer Träger | Ad 100 | Ad 100 | Ad 100 | Ad 100 |

| | AF 37 | AF 38 | AF 39 | AF 40 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Kosmetischer Träger | Ad 100 | Ad 100 | Ad 100 | Ad 100 |

| | AF 41 | AF 42 | AF 43 | AF 44 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen ¹⁾ | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Kosmetischer Träger | Ad 100 | Ad 100 | Ad 100 | Ad 100 |

| | AF 45 | AF 46 | AF 47 | AF 48 |
|---|---|---|---|---|
| Verbindung I Formel (Ia) | 0,1 bis 30,0 | 0,2 bis 20,0 | 0,3 bis 10,0 | 0,5 bis 6,0 |
| Mischung aus Cetylalkohol und Stearylalkohol | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Kosmetischer Träger | Ad 100 | Ad 100 | Ad 100 | Ad 100 |

| | AF 49 | AF 50 | AF 51 | AF 52 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Xanthan | 0,0005 bis 5,0 | 0,001 bis 1,0 | 0,005 bis 0,5 | 0,01 bis 0,3 |
| Kosmetischer Träger | Ad 100 | Ad 100 | Ad 100 | Ad 100 |

| | AF 53 | AF 54 | AF 55 | AF 56 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Kosmetischer Träger | Ad 100 | Ad 100 | Ad 100 | Ad 100 |

| | AF 57 | AF 58 | AF 59 | AF 60 |
|---|---|---|---|---|
| Verbindung I Formel (Ia) | 0,1 bis 30,0 | 0,2 bis 20,0 | 0,3 bis 10,0 | 0,5 bis 6,0 |
| Alkohol b) mit 10 bis 30 C-Atomen | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Reduktionsmittel ³⁾ | 0,05 bis 5,0 | 0,1 bis 4,0 | 0,2 bis 3,0 | 0,3 bis 2,0 |
| Kosmetischer Träger | Ad 100 | Ad 100 | Ad 100 | Ad 100 |

| | AF 61 | AF 62 | AF 63 | AF 64 |
|---|---|---|---|---|
| Verbindung I Formel (Ia) | 0,1 bis 30,0 | 0,2 bis 20,0 | 0,3 bis 10,0 | 0,5 bis 6,0 |
| Mischung aus Cetylalkohol und Stearylalkohol | 0,5 bis 18 | 1,0 bis 15 | 1,2 bis 12 | 1,5 bis 10 |
| Verbindung II Formel (IIa) | 0,1 bis 30,0 | 0,2 bis 20,0 | 0,3 bis 10,0 | 0,5 bis 6,0 |
| Xanthan | 0,0005 bis 5,0 | 0,001 bis 1,0 | 0,005 bis 0,5 | 0,01 bis 0,3 |
| Tensid ²⁾ | 0,01 bis 15 | 0,1 bis 10 | 0,2 bis 5,0 | 0,3 bis 2,0 |
| Reduktionsmittel ³⁾ | 0,05 bis 5,0 | 0,1 bis 4,0 | 0,2 bis 3,0 | 0,3 bis 2,0 |
| Kosmetischer Träger | Ad 100 | Ad 100 | Ad 100 | Ad 100 |

| | | | | |
|---|---|---|---|---|
| ¹⁾ Alkohol b) mit 10 bis 30 C-Atomen ausgewählt ist aus der Gruppe von 1-Decanol, Laurylalkohol, Myristylalkohol, Cetylalkohol, Stearylalkohol, Arachidylalkohol, Behenylalkohol, Lignocerylalkohol, Cerylalkohol, Montanylalkohol sowie deren Mischungen ²⁾ Mischung aus Cocamidopropylbetain, Natriumcetearylsulfat und PEG-40 Castor Oil ³⁾ Reduktionsmittel ist ausgewählt aus der Gruppe von Malonsäure, Oxalsäure, Oxalsäureestern, Ascorbinsäure sowie deren Mischungen, insbesondere Oxalsäure | | | | |

Die zuvor genannten besonders bevorzugten Ausführungsformen AF1 bis AF64 der erfindungsgemäßen kosmetischen Mittel zeichnen sich durch eine hervorragende Enfärbeleistung oxidativ gefärbter keratinischer Fasern aus, wobei die Entfärbung lang anhaltend ist und nicht nachdunkelt. Weiterhin führt die Anwendung dieser Entfärbeemulsionen zu einer verringerten Geruchsbelastung während der Entfärbung im Vergleich zu im Stand der Technik bekannten Entfärbemitteln.

Die kosmetischen Mittel der vorliegenden Erfindung können auch direkt vor der Anwendung aus zwei oder mehreren getrennt verpackten Zusammensetzungen hergestellt werden. Dies ist im Rahmen der vorliegenden Erfindung vorteilhaft, da die Verbindungen der Formel (I) bzw. (la) und (II) bzw. (IIa) bei alkalischen pH-Werten eine höhere Lagerstabilität besitzen, die Entfärbung jedoch bevorzugt im sauren Milieu abläuft. Aus diesen Gründen ist es von Vorteil, das für die Lagerung alkalisch eingestellte Entfärbemittel kurz vor der Anwendung auf einen sauren pH-Wert zu bringen. Die Ansäuerung des zuvor alkalisch eingestellten Entfärbemittels kurz vor der Anwendung kann durch Vermischen von zwei verschiedenen Mitteln realisiert werden, wobei das zuvor beschriebene alkalisch eingestellte erste Mittel mit einem weiteren Mittel vermischt wird, welches eine oder mehrere Säuren enthält.

Die zwei zur Herstellung des anwendungsbereiten Mittels notwendigen Komponenten werden dem Anwender zweckmäßigerweise in Form eines Kits (d.h. in Form einer Mehr-Komponenten-Verpackungseinheit) zur Verfügung gestellt, welches mindestens zwei getrennt voneinander konfektionierte Zubereitungen (1) und (2) umfasst. Die erste Zubereitung (1) beinhaltet hierbei in einem kosmetischen Träger mindestens eine Verbindung I der Formel (I) und gegebenenfalls mindestens eine Verbindung II der Formel (II), wie zuvor definiert, wohingegen die zweite Zubereitung (2) die zur Absenkung des pH-Wertes notendigen Säuren umfasst. Das anwendungsbereite Entfärbemittel wird dann durch Vermischen der Zubereitungen (1) und (2) hergestellt.

Ein zweiter Gegenstand der vorliegenden Erfindung ist daher eine Mehrkomponenten-Verpackungseinheit (Kit-of-parts) zur reduktiven Entfärbung von gefärbten keratinischen Fasern, umfassend mindestens zwei getrennt voneinander konfektionierte Zubereitungen (1) und (2), wobei
- die erste Zubereitung (1) in einem kosmetischen Träger mindestens eine Verbindung I der Formel (I) und gegebenenfalls mindestens eine Verbindung II der Formel (II), wie sie im ersten Erfindungsgegenstand definiert sind, enthält, und
- die zweite Zubereitung (2) mindestens eine anorganische und/oder organische Säure enthält, wobei die Zubereitung (1) und/oder die Zubereitung (2) mindestens einen Alkohol mit 10 bis 30 Kohlenstoffatomen in einer Gesamtmenge von 0,1 bis 20 Gew.-%, bezogen auf das Gesamtgewicht der Zubereitung (2), enthält.

Die aus der erfindungsgemäßen Verpackungseinheit herstellbaren Entfärbemittel führen zu einer verbesserten Entfärbung von gefärbten keratinischen Fasern. Weiterhin tritt nach der Entfärbung keine Nachdunklung oder Rückfärbung auf. Zudem kann mit den aus der Verpackungseinheit herstellbaren Färbemitteln die Geruchsbelastung gegenüber Entfärbemitteln des Standes der Technik deutlich gesenkt werden.

Erfindungsgemäß bevorzugt enthält die erste Zubereitung (1) einen bestimmten Wassergehalt und weist einen alkalischen pH-Wert auf, um die Stabilität der Verbindung I der Formel (I) und gegebenenfalls der Verbindung II der Formel (II) zu erhöhen. Es ist daher vorteilhaft, wenn die erste Zubereitung (1) einen pH-Wert, gemessen bei 20 °C, von pH 7,5 bis pH 12,0, vorzugsweise von pH 8,0 bis pH 11,5, bevorzugt von pH 8,5 bis pH 11,0, insbesondere von pH 9,0 bis pH 10,5, und einen Wassergehalt von 5,0 bis 99,0 Gew.-%, vorzugsweise von 15,0 bis 98,0 Gew.-%, insbesondere von 50 bis 98 Gew.-%, bezogen auf das Gesamtgewicht der Zubereitung (1), aufweist

Die zur Einstellung des pH-Wertes der ersten Zubereitung (1) verwendbaren Alkalisierungsmittel können ausgewählt sein aus der Gruppe von Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel können ausgewählt werden aus Monoethanolamin, 2-Amino-2-methylpropanol und Triethanolamin. Die als erfindungsgemäßes Alkalisierungsmittel einsetzbaren basischen Aminosäuren können ausgewählt werden aus der Gruppe, die gebildet wird aus Arginin, Lysin, Ornithin und Histidin.

Besonders bevorzugt werden ein oder mehrere Alkalisierungsmittel aus der Gruppe von Natriumhydroxid, Kaliumhydroxid, Ammoniak, Monoethanolamin und/oder Arginin verwendet. Ganz besonders bevorzugt werden Natriumhydroxid und/oder Kaliumhydroxid eingesetzt.

Es ist daher im Rahmen der vorliegenden Erfindung bevorzugt, wenn die erste Zubereitung (1) zusätzlich mindestens ein Alkalisierungsmittel aus der Gruppe von Natriumhydroxid, Kaliumhydroxid, Ammoniak, Monoethanolamin und/oder Arginin, besonders bevorzugt Natriumhydroxid und/oder Kaliumhydroxid, enthält.

Die mindestens eine Verbindung I der Formel (I) und gegebenenfalls die mindestens eine Verbindung II der Formel (II) sind bevorzugt in einer bestimmten Gesamtmenge in der ersten Zubereitung enthalten. Vorteilhafterweise enthält die Zubereitung (1) daher die mindestens eine Verbindung I der Formel (I) sowie gegebenenfalls die mindestens eine Verbindung II der Formel (II) in einer Gesamtmenge von 0,6 bis 60 Gew.-%, vorzugsweise von 0,9 bis 50 Gew.-%, bevorzugt von 1,5 bis 40 Gew.-%, insbesondere von 2,0 bis 30 Gew.-%, bezogen auf das Gesamtgewicht der Zubereitung (1). Unter Gesamtmenge wird hierbei die Menge aller in der Zusammensetzung (1) enthaltenen Verbindungen I der Formel (I) sowie gegebenenfalls aller Verbindungen II der Formel (II) verstanden.

Die zweite Zubereitung (2) weist bevorzugt einen sauren pH-Wert auf, um die Einstellung eines sauren pH-Wertes des aus der Verpackungseinheit herstellbaren Entfärbemittels zu gewährleisten. Aufgrund der Instabilitäten der Reduktionsmittel im sauren Milieu enthält die zweite Zubereitung (2) daher bevorzugt keine Verbindung I der Formel (I), keine Verbindung II der Formel (II) sowie keine unter dem ersten Erfindungsgegenstand angeführten weiteren Reduktionsmittel. Zur Einstellung eines sauren pH-Werts enthält die zweite Zubereitung (2) mindestens eine Säure. Vorteilhafterweise werden im Rahmen der vorliegenden Erfindung organische Säuren eingesetzt. Im Rahmen der vorliegenden Erfindung ist es daher bevorzugt, wenn die zweite Zubereitung (2) mindestens eine organische Säure, ausgewählt aus der Gruppe von Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure), Oxalessigsäureester, 1-Hydroxyethan-1,1-diphosphonsäure sowie deren Mischungen, enthält.

Weiterhin ist es bevorzugt, wenn die zweite Zubereitung (2) einen pH-Wert, gemessen bei 20 °C, von pH 0,1 bis pH 4,0, vorzugsweise von pH 0,2 bis pH 3,0, insbesondere von pH 0,5 bis pH 2,0, und einen Wassergehalt von 5,0 bis 99 Gew.-%, bezogen auf das Gesamtgewicht der zweiten Zubereitung (2), aufweist.

Eine besonders hohe Verminderung der Geruchsbelastung wird erhalten, wenn als zweite Zubereitung (2) eine Emulsion eingesetzt wird. Es ist daher erfindungsgemäß bevorzugt, dass die zweite Zubereitung (2) in Form einer Emulsion vorliegt.

Zur Stabilisierung der Emulsion ist es von Vorteil, wenn die zweite Zubereitung (2) mindestens ein Tensid aus der Gruppe der anionischen Tenside, der amphoteren und/oder zwitterionischen Tenside, der nichtionischen Tenside, der kationischen Tenside sowie deren Mischungen enthält. Bezüglich der einsetzbaren Tenside wird auf obige Ausführungen zu dem ersten Erfindungsgegenstand verwiesen. Die anionischen, amphoteren bzw. zwitterionischen, nichtionischen und kationischen Tenside können jeweils in einer Gesamtmenge von 0,01 bis 40 Gew.-%, vorzugsweise von 0,1 bis 30 Gew.-%, bevorzugt von 0,2 bis 20 Gew.-%, insbesondere von 0,3 bis 10 Gew.-%, bezogen auf das Gesamtgewicht der zweiten Zubereitung (2), enthalten sein.

Wie bereits zuvor beschrieben wird das anwendungsbereite Entfärbemittel bevorzugt durch Vermischen von zwei Zubereitungen (1) und (2) hergestellt. Prinzipiell können die Zubereitungen (1) und (2) hierbei in verschiedenen Mischungsverhältnissen, wie beispielsweise (1)/(2) von 20:1 bis 1:20, vermischt werden. Bevorzugt werden die Zubereitungen (1) und (2) in einem Mischungsverhältnis von 1:10 bis 10:1, besonders bevorzugt von 1:4 bis 4:1 miteinander vermischt. Die Mischungsverhältnisse geben hierbei das Verhältnis der Gewichtsmengen der Zubereitungen (1) und (2) zueinander an.

Die Zubereitungen (1) und (2) werden getrennt voneinander konfektioniert und können jeweils in jedem hierfür geeigneten Behältnis konfektioniert vorliegen. Geeignete Behältnisse sind beispielsweise Glas- oder insbesondere Kunststoff-Flaschen, Dosen, Tuben oder auch andere geeignete Container.

Zur Herstellung der anwendungsbereiten Mischung kann beispielsweise die erste Zubereitung (1) aus Container (C1) vollständig in Container (C2) - welcher bereits die zweite Zubereitung (2) enthält - überführt werden. In diesem Fall wird die Größe des Containers (C2) so gewählt, dass der Container (C2) die Gesamtmenge der Zubereitungen (1) und (2) aufnehmen kann und auch ein Vermischen der beiden Zubereitungen (1) und (2), z.B. durch Schütteln oder Verrühren, zulässt.

Analog kann die Herstellung der Mischung auch durch vollständige Überführung der zweiten Zubereitung (2) aus Container (C2) in Container (C1) - welcher bereits die erste Zubereitung (1) enthält - erfolgen. In diesem Fall sollte die Größe des Containers (C1) so gewählt werden, dass der Container (C1) die Gesamtmenge der Zubereitungen (1) und (2) aufnehmen kann und auch ein Vermischen der beiden Zubereitungen (1) und (2), z.B. durch Schütteln, oder Verrühren, zulässt.

Eine weitere Möglichkeit zur Herstellung der Anwendungsmischung ist die vollständige Überführung beider Zubereitungen (1) und (2) aus den Containern (C1) und (C2) in ein drittes Behältnis, welches dann das Vermischen beider Mittel - z.B. durch Schütteln, oder Verrühren - erlaubt.

Die Mehrkomponentenverpackungseinheit (Kit-of-parts) kann auch noch eine weitere dritte, getrennt konfektionierte Zubereitung (3) umfassen. Dies ist insbesondere dann der Fall, wenn die Zubereitung (1), welche die Verbindung I der Formel (I) und gegebenenfalls die Verbindung II der Formel (II) enthält, wasserfrei konfektioniert werden soll.

In diesem Fall wird die Verbindung I der Formel (I) und gegebenenfalls die Verbindung II der Formel (II) enthaltende Zubereitung (1) zunächst mit der wässrigen Zubereitung (3) vermischt - dieser Vermischungsvorgang gewährleistet die vollständige Lösung der Verbindungen I der Formel (I) sowie gegebenenfalls der Verbindungen II der Formel (II)). Zur Herstellung der finalen Anwendungsmischung wird die Mischung der Zubereitungen (1) und (3) anschließend mit der Zubereitung (2) vermischt und hierdurch der für die Anwendung optimale pH-Wert eingestellt.

In diesem Fall ist eine besonders bevorzugte Mehrkomponenten-Verpackungseinheit (Kit-of-parts) dadurch gekennzeichnet, dass
- die erste Zubereitung (1) einen Wassergehalt von 0 bis 5,0 Gew.-%, vorzugsweise von 0 bis 2,5 Gew.-%, bevorzugt von 0 bis 1,0 Gew.-%, insbesondere von 0 bis 0,1 Gew.-%, bezogen auf das Gesamtgewicht der ersten Zubereitung (1) besitzt und
- die Mehrkomponenten-Verpackungseinheit mindestens eine weitere getrennt von den Zubereitungen (1) und (2) konfektionierte Zubereitung (3) umfasst, wobei die dritte Zubereitung (3) - bezogen auf das Gesamtgewicht der Zubereitung (3) - einen Wassergehalt von 5,0 bis 99,0 Gew.-%, insbesondere von 15,0 bis 85,0 Gew.-%, aufweist.

Die Zubereitungen (1), (2) sowie gegebenenfalls (3) können weiterhin zusätzliche Wirk-, Hilfs- und Zusatzstoffe enthalten, um die Entfärbeleistung zu verbessern und weitere gewünschte Eigenschaften der Mittel einzustellen. Beispielsweise kann eine oder mehrere der Zubereitungen zusätzlich nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben der Zubereitungen; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft, enthalten. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, welche das entsprechende Wissen des Fachmannes wiedergeben.

Bezüglich weiterer bevorzugter Ausführungsformen der erfindungsgemäßen Mehrkomponenten-Verpackungseinheit (Kit-of-Parts) gilt mutatis mutandis das zu den erfindungsgemäßen Mitteln Gesagte.

Die erfindungsgemäßen, zuvor beschriebenen Mittel und Mehrkomponenten-Verpackungseinheiten (Kit-of-Parts) lassen sich in Verfahren zur Färbung und reduktiven Entfärbung von keratinischen Fasern, insbesondere menschlichen Haaren, einsetzen.

Ein dritter Gegenstand der vorliegenden Erfindung ist daher ein Verfahren zur Färbung und reduktiven Entfärbung von keratinischen Fasern, insbesondere menschlichen Haaren, umfassend die folgenden Verfahrensschritte in der angegebenen Reihenfolge
a) Applizierung eines kosmetischen Färbemittels, das mindestens einen direktziehenden Farbstoff und/oder mindestens ein Oxidationsfarbstoffvorprodukt enthält, auf die keratinischen Fasern
b) Einwirkenlassen des Färbemittels für einen Zeitraum von 5 bis 60 Minuten
c) Ausspülen des Färbemittels
d) Applizierung eines erfindungsgemäßen kosmetischen Mittels oder eines aus einem erfindungsgemäßen Kit-of-parts hergestellten Entfärbemittels auf die keratinischen Fasern,
e) Einwirkenlassen des Entfärbemittels bei 15 bis 45 °C für einen Zeitraum von 5 bis 60 Minuten, vorzugsweise von 10 bis 55 Minuten, bevorzugt von 15 bis 55 Minuten, insbesondere von 20 bis 50 Minuten,
f) Ausspülen des Entfärbemittels,
g) gegebenenfalls Applizierung eines Nachbehandlungsmittels auf die keratinischen Fasern, wobei das Nachbehandlungsmittel mindestens ein Tensid aus der Gruppe von anionischen Tensiden, kationischen Tensiden, nichtionischen Tensiden, amphoteren und/oder zwitterionischen Tensiden sowie deren Mischungen enthält.

Das erfindungsgemäße Verfahren zur Färbung und reduktiven Entfärbung keratinischer Fasern resultiert in einer verbesserten Entfärbung und einer verringerten Geruchsbelastung gegenüber Verfahren des Standes der Technik.

Die Schritte a), b) und c) des Verfahrens stellen den Färbevorgang der Keratinfasern dar und werden demzufolge in direkter zeitlicher Abfolge hintereinander ausgeführt. Für die Abfolge der Schritte c) und d) besteht prinzipiell keine zeitliche Limitierung. So kann Schritt d) Stunden, Tage oder auch beispielsweise bis zu sechs Wochen nach Beendigung des Schrittes c) erfolgen.

Das Verfahren soll jedoch das unerwünschte Farbergebnis des Färbevorgangs der Schritte a) bis c) entfernen, daher versteht es sich von selbst, dass die Entfärbung nur dann vorgenommen werden kann, wenn die gefärbten Fasern auch noch das unerwünschtes Farbergebnis zeigen. Wurden die Keratinfasern beispielsweise mit direktziehenden Farbstoffen gefärbt und hat sich diese Färbung nach 2 Wochen vollständig ausgewaschen, so ist ein im Anschluss daran stattfindender Entfärbevorgang weder notwendig noch erfindungsgemäß.

In Schritt d) des erfindungsgemäßen Verfahrens wird ein kosmetisches Mittel des ersten Erfindungsgegenstands oder ein aus dem erfindungsgemäßen Kits-of-parts hergestelltes Mittel auf die keratinischen Fasern appliziert.

Die Schritte d), e) und f) des Verfahrens stellen den Entfärbevorgang der Keratinfasern dar und werden demzufolge wieder in direkter zeitlicher Abfolge hintereinander ausgeführt.

Schritt g) des Verfahrens, d.h. die Applizierung eines Nachbehandlungsmittels, ist optional. Für die Abfolge der Schritte f) und des optionalen Schrittes g) bestehen wieder keine zeitlichen Limitierungen. Es ist jedoch von Vorteil, wenn die Nachbehandlung des Schrittes g) maximal zwei Tage nach Abschluss des Schrittes f) erfolgt. Der Nachbehandlungsschritt g) kann auch öfter als einmal wiederholt werden, beispielsweise wenn es sich bei dem Nachbehandlungsmittel um ein Shampoo handelt.

Die Auftragung der Färbezubereitung und der erfindungsgemäßen Entfärbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel und Entfärbezubereitung mit einer Applikationshilfe auf die keratinischen Fasern aufzutragen.

Bezüglich des erfindungsgemäßen kosmetischen Mittels, des aus dem erfindungsgemäßen Kit-of-parts hergestellten Mittels sowie weiterer bevorzugter Ausführungsformen des erfindungsgemäßen Verfahrens gilt mutatis mutandis das zu den erfindungsgemäßen kosmetischen Mitteln sowie zu der erfindungsgemäßen Verpackungseinheit Gesagte.

Darüber hinaus ist ein weiterer Gegenstand der vorliegenden Erfindung die Verwendung eines erfindungsgemäßen kosmetischen Mittels oder eines aus einem erfindungsgemäßen Kit-of-parts hergestellten kosmetischen Mittels zur Entfärbung von gefärbten keratinischen Fasern, insbesondere menschlicher Haare. Der Einsatz dieser Mittel führt zu einer verbesserten Entfärbung von gefärbten keratinischen Fasern und vermeidet eine Nachdunklung oder Rückfärbung. Weiterhin wird die Geruchsbelastung während der Entfärbung gegenüber Mitteln des Standes der Technik vermindert.

Schließlich ist ein weiterer Gegenstand der vorliegenden Erfindung die Verwendung eines erfindungsgemäßen kosmetischen Mittels oder eines aus einem erfindungsgemäßen Kit-of-parts hergestellten kosmetischen Mittels zur Verringerung der Geruchsbelastung während der Entfärbung von gefärbten keratinischen Fasern.

Bezüglich weiterer bevorzugter Ausführungsformen der erfindungsgemäßen Verwendungen gilt mutatis mutandis das zu den erfindungsgemäßen kosmetischen Mitteln sowie zu der erfindungsgemäßen Verpackungseinheit Gesagte.

Die nachfolgenden Beispiele sollen bevorzugte Ausführungsformen der Erfindung erläutern, ohne sie jedoch einzuschränken.

### Beispiele:

### 1.1. Färbung

Es wurden die folgenden Formulierungen hergestellt (alle Angaben in Gew.-%):

**Färbecreme (F1)**

| Rohstoff | Gew.% |
|---|---|
| Cetearylalkohol | 6,6 |
| C12-C18 Fettalkohole | 2,4 |
| Ceteareth-20 | 0,6 |
| Ceteareth-12 | 0,6 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19 - 21 %ige wässrige Lösung) | 3,8 |
| Natriumhydroxid | 0,26 |
| p-Toluylendiamin, Sulfat | 0,48 |
| m-Aminophenol | 0,02 |
| 4-Chlorresorcin | 0,09 |
| 2-Methylresorcin | 0,04 |
| Resorcin | 0,12 |
| Ammniumsulfat | 0,71 |
| Natriumsulfit | 0,4 |
| Ascorbinsäure | 0,1 |
| 1-Hydroxyethan-1,1-diphosphonsäure (60 %ige wässrige Lösung) | 0,2 |
| Natriumwasserglas | 0,5 |
| L-Serin | 1,0 |
| Ammoniak (25 %ige wässrige Lösung) | 6,7 |
| Wasser | ad 100 |

**Färbecreme (F2)**

| Rohstoff | Gew.% |
|---|---|
| Cetearylalkohol | 8,5 |
| C12-C18 Fettalkohole | 2,4 |
| Ceteareth-20 | 0,6 |
| Ceteareth-12 | 0,6 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19 - 21 %ige wässrige Lösung) | 3,8 |
| Kaliumhydroxid | 0,83 |
| p-Toluylendiamin, Sulfat | 0,89 |
| m-Aminophenol | 0,04 |
| 2-Methylresorcin | 0,10 |
| Resorcin | 0,17 |
| 4-Chlorresorcin | 0,08 |
| 2-Am i no-3-hyd roxypyrid in | 0,03 |
| 2,7-Dihydroxynaphthalin | 0,09 |
| Glycin | 1,0 |
| Natriumsulfit | 0,4 |
| Ascorbinsäure | 0,1 |
| 1-Hydroxyethan-1,1-diphosphonsäure (60 %ige wässrige Lösung) | 0,2 |
| Natriumwasserglas | 0,5 |
| Marulaöl | 0,6 |
| Monoethanolamin | 5,0 |
| Wasser | Ad 100 |

**Oxidationsmittel (Ox)**

| Rohstoff | Gew.% |
|---|---|
| Natriumbenzoat | 0,04 |
| Dipicolinsäure | 0,1 |
| Dinatriumpyrophosphat | 0,1 |
| Kaliumhydroxid | 0,09 |
| 1,2-Propylenglycol | 1,0 |
| 1-Hydroxyethan-1,1-diphosphonsäure (60 %ige wässrige Lösung) | 0,25 |
| Paraffinum Liquidum | 0,30 |
| Steartrimoniumchlorid | 0,39 |
| Cetearylalkohol | 3,4 |
| Ceteareth-20 | 1,0 |
| Wasserstoffperoxid (50 %ige wässrige Lösung) | 12,0 |

Haarsträhnen (Kerling Euronaturhaar weiß) wurden farbmetrisch vermessen, es wurde jeweils der L-Wert (als Maß für die Helligkeit der Haarsträhne) bestimmt.

Dann wurden die Farbcremes (F1 und F2) und das Oxidationsmittel (Ox) jeweils im Mengenverhältnis 1:1 miteinander vermischt und auf Haarsträhnen (Kerling Euronaturhaar weiß) appliziert.

Das Gewichtsverhältnis Anwendungsmischung : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen.

Danach wurden die Haarsträhnen erneut farbmetrisch vermessen, es wurde wieder der L-Wert (als Maß für die Helligkeit der Haarsträhne) bestimmt.

Die Strähnen waren in einem dunkelblonden Farbton (F1 + OX) bzw. in einem mokkabraunen Farbton (F2 + Ox) gefärbt.

### 1.2 Entfärbung

Es wurden die folgenden Entfärbemittel hergestellt (alle Angaben in Gew.-%):

**Zubereitungen (1) mit pH-Wert 6,0 - 10,5**

| **Rohstoff** | **Z1-1** | **Z1-2** | **Z1-3** | **Z1-4** |
|---|---|---|---|---|
| Xanthan Gum | 0,8 | 0,8 | 0,6 | 0,6 |
| Propylengylcol | 1,6 | 1,6 | 1,2 | 1,2 |
| Cocamidopropylbetain (40%ig) | 4,0 | 4,0 | 3,0 | 3,0 |
| Cyclanon Eco (wässrige Lösung der Verbindung der Formel (Ia) und (IIa) im Molverhältnis 1:1) | 20 | -- | -- | 40 |
| Natriumdithionit | -- | 20 | 40 | - |
| Wasser | Ad 100 | Ad 100 | Ad 100 | Ad 100 |

**Zubereitungen (2) mit pH-Wert 0,5 - 2,0**

| **Rohstoff** | **Z2-1 (Gel)** | **Z2-2 (Emulsion)** |
|---|---|---|
| Xanthan Gum | 0,9 | -- |
| Propylengylcol | 1,8 | -- |
| Cocamidopropylbetain (40%ig) | 4,4 | -- |
| Schwefelsäure (20%ig) | 12 | 5,3 |
| Oxalessigsäure | -- | 0,67 |
| Emulgade FT ¹⁾ | -- | 2,8 |
| Wasser | Ad 100 | Ad 100 |

| | | |
|---|---|---|
| ¹⁾ 77 Gew.-% Cetearylalkohol, 15 Gew.-% PEG-40 Castor Oil, 7,5 Gew.-% Natriumcetearylsulfat (INCI: CETEARYL ALCOHOL, PEG-40 CASTOR OIL, SODIUM CETEARYL SULFATE; BASF) | | |

**Zubereitungen (3)**

| **Rohstoff** | **Z3-1** | **Z3-2** |
|---|---|---|
| Xanthan Gum | 0,9 | 0,9 |
| Propylengylcol | 1,8 | 1,8 |
| Cocamidopropylbetain (40%ig) | 4,5 | 4,5 |
| Cyclanon Eco (wässrige Lösung der Verbindung der Formel (Ia) und (IIa) im Molverhältnis 1:1) | 10 | -- |
| Natriumdithionit | -- | 10 |
| Schwefelsäure, 20% ig | Ad pH 1,8 | Ad pH 1,8 |
| Wasser | Ad 100 | Ad 100 |

Zur Herstellung der anwendungsbereiten Entfärbemittel wurden jeweils die Zubereitungen Z1-1 bis Z1-4 mit einer der Zubereitungen Z2-1 oder Z2-2 vermischt. Die Zubereitungen Z3-1 und Z3-2 wurden direkt nach der Herstellung als Entfärbemittel verwendet. Alle Entfärbemittel wiesen einen pH-Wert von 1,5 bis 3,5 auf.

| Entfärbemittel | Hergestellt aus |
|---|---|
| 1 | Z1-1 + Z2-1 im Verhältnis 1:1 |
| 2 | Z1-2 + Z2-1 im Verhältnis 1:1 |
| 3 | Z1-3 + Z2-2 im Verhältnis 1:3 |
| 4* | Z1-4 + Z2-2 im Verhältnis 1:3 |
| 5 | Z3-1 |
| 6 | Z3-2 |

| | |
|---|---|
| * erfindungsgemäß | |

Das erfindungsgemäße Entfärbemittel 4 wurde auf das Haar aufgetragen, wobei das Gewichtsverhältnis Anwendungsmischung : Haar 4:1 betrug. Nach einer Einwirkzeit von 30 Minuten bei einer Temperatur von 32 Grad Celsius wurden die Strähnen mit Wasser gespült, getrocknet und mindestens 24 Stunden bei Raumtemperatur ruhen gelassen.

Danach wurden die Haarsträhnen erneut farbmetrisch vermessen, es wurde wieder der L-Wert (als Maß für die Helligkeit der Haarsträhne) bestimmt.

Die Beurteilung des Entfärbeergebnisses erfolgte anhand der Bestimmung des ΔL-Wertes ΔL = L(nach der Entfärbung) - L(vor der Entfärbung)

Je höher der ΔL-Wert ist, desto besser wurden die gefärbten Haarsträhnen entfärbt.

| F1 + OX | L-Wert | ΔL-Wert |
|---|---|---|
| coloriertes Haar | 33,1 | 23,8 |
| entfärbtes Haar | 56,9 | |
| | | |

| F2 + OX | L-Wert | ΔL-Wert |
|---|---|---|
| coloriertes Haar | 25,7 | 25,5 |
| entfärbtes Haar | 51,2 | |

Sowohl die mit F1+OX gefärbte Strähne als auch die mit F2+OX gefärbte Strähne war nach Anwendung des erfindungsgemäßen Entfärbemittels 4 in einem signifikanten Ausmaß entfärbt.

Weiterhin wurde die Geruchsbelastung während der Entfärbung bestimmt. Hierfür wurden jeweils 4 g des jeweiligen Entfärbemittels pro 1 g oxidativ gefärbter Haarsträhne verwendet. Nach 5 Minuten bei 32 °C wurde die Geruchsbelastung während der Entfärbung bestimmt und mittels Notensystem (1 = sehr leichte Geruchsbelastung, 2 = leichte Geruchsbelastung, 3 = mittlere Geruchsbelastung, 4 = starke Geruchsbelastung) bewertet. Die Ergebnisse sind in nachfolgender Tabelle dargestellt:

| Entfärbemittel | Geruchsbelastung |
|---|---|
| 1 | 2 |
| 2 | 4 |
| 3 | 3 |
| 4* | 1 |
| 5 | 3 |
| 6 | 4 |

| | |
|---|---|
| * erfindungsgemäß | |

Durch die Verwendung einer Emulsion, welche einen Alkohol mit 10 bis 30 Kohlenstoffatomen enthält, in Kombination mit Reduktionsmitteln der Formel (la) und (IIa) konnte die Geruchsbelastung während der Entfärbung signifikant verbessert werden.

## Patentansprüche

1. Kosmetisches Mittel zur reduktiven Entfärbung von gefärbten keratinischen Fasern, insbesondere menschlichen Haaren, enthaltend in einem kosmetischen Träger
a) mindestens eine Verbindung I der Formel (I)
A[(CR¹R²)SO₂M]_{p,q} (I)
in welcher
A für N(R³)_{3-q} oder O(R⁴)₂₋ₚ steht
R¹, R², R⁴ unabhängig voneinander für ein Wasserstoffatom oder für eine C₁-C₆-Alkylgruppe stehen,
R³ für identische oder verschiedene Reste ausgewählt aus einem Wasserstoffatom, einer C₁-C₂₀-Alkylgruppe, einer C₃-C₈-Cycloalkylgruppe ggf. substituiert durch ein bis drei C₁-C₄-Alkylreste steht
M für identische oder verschiedene Reste ausgewählt aus einem Wasserstoffatom oder einem Äquivalent eines Alkali-, Erdalkali- oder Metallions, insbesondere für Natrium, Kalium, ½ Magnesium, ½ Calcium, ½ Zink, oder für ein Ammoniumion (NH₄⁺) steht,
p für die Zahlen 1 oder 2 steht,
q für die Zahlen 1, 2 oder 3 steht,
wobei
- mindestens einer der Reste R¹, R², R⁴ für eine C₁-C₆-Alkylgruppe steht, wenn A für O(R⁴₂₋ₚ) steht, und
- R³ nicht für ein Wasserstoffatom steht, wenn q gleich 1 ist, und
b) mindestens einen Alkohol mit 10 bis 30 Kohlenstoffatomen in einer Gesamtmenge von 0,1 bis 20 Gew.-%, bezogen auf das Gesamtgewicht des kosmetischen Mittels,
**dadurch gekennzeichnet, dass** es mindestens eine Verbindung I der Formel (la)
N(CH₂SO₂Na)₃ (Ia)
enthält.

2. Kosmetisches Mittel nach Anspruch 1, **dadurch gekennzeichnet, dass** die mindestens eine Verbindung I der Formel (I) in einer Gesamtmenge von 0,1 bis 30,0 Gew.-%, vorzugsweise von 0,2 bis 20,0 Gew.-%, bevorzugt von 0,3 bis 10,0 Gew.-%, insbesondere von 0,5 bis 6,0 Gew.-%, bezogen auf das Gesamtgewicht des kosmetischen Mittels, enthalten ist.

3. Kosmetisches Mittel nach einem der vorangehenden Ansprüche, **dadurch gekennzeichnet, dass** der mindestens eine Alkohol b) mit 10 bis 30 Kohlenstoffatomen ausgewählt ist aus der Gruppe von 1-Decanol, Laurylalkohol, Myristylalkohol, Cetylalkohol, Stearylalkohol, Arachidylalkohol, Behenylalkohol, Lignocerylalkohol, Cerylalkohol, Montanylalkohol sowie deren Mischungen, insbesondere aus einer Mischung von Cetylalkohol und Stearylalkohol.

4. Kosmetisches Mittel nach einem der vorangehenden Ansprüche, **dadurch gekennzeichnet, dass** der mindestens eine Alkohol b) in einer Gesamtmenge von 0,5 bis 18 Gew.-%, vorzugsweise von 1,0 bis 15 Gew.-%, bevorzugt von 1,2 bis 12 Gew.-%, insbesondere von 1,5 bis 10 Gew.-%, bezogen auf das Gesamtgewicht des kosmetischen Mittels, enthalten ist.

5. Kosmetisches Mittel nach einem der vorangehenden Ansprüche, **dadurch gekennzeichnet, dass** es zusätzlich eine Verbindung II der Formel (II)
A[(CR¹R²)SO₃M]_{p,q} (II)
in welcher
A für N(R³)_{3-q} oder O(R⁴)₂₋ₚ steht
R¹, R², R⁴ unabhängig voneinander für ein Wasserstoffatom oder für eine C₁-C₆-Alkylgruppe stehen,
R³ für identische oder verschiedene Reste ausgewählt aus einem Wasserstoffatom, einer C₁-C₂₀-Alkylgruppe, einer C₃-C₈-Cycloalkylgruppe ggf. substituiert durch ein bis drei C₁-C₄-Alkylreste steht
M für identische oder verschiedene Reste ausgewählt aus einem Wasserstoffatom oder einem Äquivalent eines Alkali-, Erdalkali- oder Metallions, insbesondere für Natrium, Kalium, ½ Magnesium, ½ Calcium, ½ Zink, oder für ein Ammoniumion (NH₄⁺) steht,
p für die Zahlen 1 oder 2 steht,
q für die Zahlen 1, 2 oder 3 steht,
wobei
- mindestens einer der Reste R¹, R², R⁴ für eine C₁-C₆-Alkylgruppe steht, wenn A für O(R⁴₂₋ₚ) steht, und
- R³ nicht für ein Wasserstoffatom steht, wenn q gleich 1 ist,
**dadurch gekennzeichnet, dass** es mindestens eine Verbindung II der Formel (IIa)
N(CH₂SO₃Na)₃ (IIa)
enthält.

6. Kosmetisches Mittel nach Anspruch 5, **dadurch gekennzeichnet, dass** die mindestens eine Verbindung II der Formel (II) in einer Gesamtmenge von 0,1 bis 30,0 Gew.-%, vorzugsweise von 0,2 bis 20,0 Gew.-%, bevorzugt von 0,3 bis 10,0 Gew.-%, insbesondere von 0,5 bis 6,0 Gew.-%, bezogen auf das Gesamtgewicht des kosmetischen Mittels, enthalten ist.

7. Kosmetisches Mittel nach einem der vorangehenden Ansprüche, **dadurch gekennzeichnet, dass** es als Emulsion vorliegt.

8. Mehrkomponenten-Verpackungseinheit (Kit-of-parts) zur reduktiven Entfärbung von gefärbten keratinischen Fasern, umfassend mindestens zwei getrennt voneinander konfektionierte Zubereitungen (1) und (2), wobei
- die erste Zubereitung (1) in einem kosmetischen Träger mindestens eine Verbindung I der Formel (I) gemäß Anspruch 1 und gegebenenfalls mindestens eine Verbindung II der Formel (II) gemäß Anspruch 5 enthält, und
- die zweite Zubereitung (2) mindestens eine anorganische und/oder organische Säure enthält,
wobei die Zubereitung (1) und/oder die Zubereitung (2) mindestens einen Alkohol mit 10 bis 30 Kohlenstoffatomen in einer Gesamtmenge von 0,1 bis 20 Gew.-%, bezogen auf das Gesamtgewicht der Zubereitung (2), enthält.

9. Mehrkomponenten-Verpackungseinheit (Kit-of-parts) nach Anspruch 8, **dadurch gekennzeichnet, dass** die zweite Zubereitung (2) mindestens eine organische Säure, ausgewählt aus der Gruppe von Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure), Oxalessigsäureester, 1-Hydroxyethan-1,1-diphosphonsäure sowie deren Mischungen, enthält.

10. Mehrkomponenten-Verpackungseinheit (Kit-of-parts) nach einem der Ansprüche 8 oder 9, **dadurch gekennzeichnet, dass** die zweite Zubereitung (2) als Emulsion vorliegt.

11. Verfahren zur Färbung und reduktiven Entfärbung von keratinischen Fasern, insbesondere menschlichen Haaren, umfassend die folgenden Verfahrensschritte in der angegebenen Reihenfolge
(a) Applizierung eines kosmetischen Färbemittels, das mindestens einen direktziehenden Farbstoff und/oder mindestens ein Oxidationsfarbstoffvorprodukt enthält, auf die keratinischen Fasern
(b) Einwirkenlassen des Färbemittels für einen Zeitraum von 5 bis 60 Minuten
(c) Ausspülen des Färbemittels
(d) Applizierung eines Entfärbemittels nach einem der Ansprüche 1 bis 7 oder eines aus einem Kit-of-parts nach einem der Ansprüche 8 bis 10 hergestellten Entfärbemittels auf die keratinischen Fasern,
(e) Einwirkenlassen des Entfärbemittels bei 20 bis 45 °C für einen Zeitraum von 5 bis 60 Minuten, vorzugsweise von 10 bis 55 Minuten, bevorzugt von 15 bis 55 Minuten, insbesondere von 20 bis 50 Minuten,
(f) Ausspülen des Entfärbemittels,
(g) gegebenenfalls Applizierung eines Nachbehandlungsmittels auf die keratinischen Fasern, wobei das Nachbehandlungsmittel mindestens ein Tensid aus der Gruppe von anionischen Tensiden, kationischen Tensiden, nichtionischen Tensiden, amphoteren und/oder zwitterionischen Tensiden sowie deren Mischungen enthält.

12. Verwendung eines kosmetischen Mittels nach einem der Ansprüche 1 bis 7 oder eines aus einem Kit-of-parts nach einem der Ansprüche 8 bis 10 hergestellten kosmetischen Mittels zur Entfärbung von gefärbten keratinischen Fasern, insbesondere menschlicher Haare.

13. Verwendung eines kosmetischen Mittels nach einem der Ansprüche 1 bis 7 oder eines aus einem Kit-of-parts nach einem der Ansprüche 8 bis 10 hergestellten kosmetischen Mittels zur Verringerung der Geruchsbelastung während der Entfärbung von gefärbten keratinischen Fasern.

## Claims

1. Cosmetic agent for the reductive decoloration of dyed keratinous fibers, more particularly human hair, containing in a cosmetic carrier
a) at least compound of I of the Formula (I)
A[(CR¹R²)SO₂M]_{p,q} (I)
wherein
A denotes N(R³)_{3-q} or O(R⁴)₂₋ₚ
R¹, R², R⁴ denote independently a hydrogen atom or a C₁-C₆-alkyl group,
R³ denotes identical or various radicals selected from a hydrogen atom, a C₁-C₂₀ alkyl group, a C₃-C₈ cycloalkyl group, substituted by one to three C₁-C₄ alkyl radicals if necessary
M denotes identical or various residues selected from a hydrogen atom or an equivalent of an alkali, alkali earth or metal ions, more particularly for sodium, potassium, ½ magnesium, ½ calcium, ½ zinc, or an ammonium ion (NH₄⁺),
p denotes the integer 1 or 2,
q denotes the integer 2 or 3,
wherein
- at least one of the radicals R¹, R², R⁴ denotes a C₁-C₆ alkyl group, if A denotes O(R⁴₂₋ₚ), and
- R³ does not denote a hydrogen atom if q is equal to 1, and
b) at least one alcohol with 10 to 30 hydrogen atoms in a total quantity of 0.1 to 20 wt.%, relative to the total weight of the cosmetic agent,
**characterized in that** it contains at least one compound I of the Formula (la)
N(CH₂SO₂Na)₃ (Ia).

2. Cosmetic agent according to claim 1, **characterized in that** the cosmetic agent contains the at least one compound I of the Formula (I) in a total quantity of 0.1 to 30.0 wt.%, preferably 0.2 to 20.0 wt.%, more preferably 0.3 to 10.0 wt.%, and most preferably 0.5 to 6.0 wt.%, relative to the total weight of the cosmetic agent.

3. Cosmetic agent according to one of the preceding claims, **characterized in that** the at least one alcohol b) has 10 to 30 carbon atoms selected from the group of 1-decanol, lauryl alcohol, myristyl alcohol, cetyl alcohol, stearyl alcohol, arachylalcohol and behenyl alcohol, lignoceryl alcohol, ceryl alcohol, montanyl alcohol, as well as the mixtures thereof, more particularly from a mixture of cetyl alcohol and stearyl alcohol

4. Cosmetic agent according to one of the preceding claims, **characterized in that** the at least one alcohol is contained in a total quantity of 0.5 to 18 wt.%, preferably 1.0 to 15 wt.%, more preferably 1.2 to 12 wt.%, and most preferably 1.5 to 10 wt.%, relative to the total weight of the cosmetic agent.

5. Cosmetic agent according to one of the preceding claims, **characterized in that** it contains in addition a compound II of the Formula (II)
A[(CR¹R²)SO₃M]_{p,q} (II)
wherein
A denotes N(R³)_{3-q} or O(R⁴)₂₋ₚ
R¹, R², R⁴ denote independently a hydrogen atom or a C₁-C₆-alkyl group,
R³ denotes identical or various radicals selected from a hydrogen atom, a C₁-C₂₀ alkyl group, a C₃-C₈ cycloalkyl group, substituted by one to three C₁-C₄ alkyl radicals if necessary
M denotes identical or various residues selected from a hydrogen atom or an equivalent of an alkali, alkali earth or metal ions, more particularly for sodium, potassium, ½ magnesium, ½ calcium, ½ zinc, or an ammonium ion (NH₄⁺),
p denotes the integer 1 or 2,
q denotes the integer 2 or 3,
wherein
- at least one of the radicals R¹, R², R⁴ denotes a C₁-C₆ alkyl group, if A denotes O(R⁴₂₋ₚ), and
- R³ does not denote a hydrogen atom if q is equal to 1,
**characterized in that** it contains at least one compound II of the Formula (IIa)
N(CH₂SO₃Na)₃ (IIa).

6. Cosmetic agent according to claim 5, **characterized in that** the at least one compound II of the Formula (II) is contained in a total quantity of 0.1 to 30.0 wt.%, preferably 0.2 to 20.0 wt.%, more preferably 0.3 to 10.0 wt.%, and most preferably 0.5 to 6.0 wt.%, relative to the total weight of the cosmetic agent.

7. Cosmetic agent according to one of the preceding claims, **characterized in that** it exists in the form of an emulsion.

8. Kit-of-parts for the reductive decoloration of dyed keratinous fibers, comprising at least two preparations (1) and (2) packaged separately from one another, wherein
- the first preparation (1) contains in a cosmetic carrier at least one compound I of the Formula (I) according to claim 1 and optionally at least one compound II of the Formula (II) according to claim 5, and
- the second preparation (2) contains at least one inorganic and/or organic acid,
wherein preparation (1) and/or preparation (2) contains at least one alcohol with 10 to 30 carbon atoms in a total quantity of 0.1 to 20 wt.%, relative to the total weight of preparation (2).

9. Kit-of-parts according to claim 8, **characterized in that** the second preparation (2) contains at least one organic acid, selected from the group of citric acid, tartaric acid, malic acid, lactic acid, acetic acid, sulphuric acid, hydrochloric acid, phosphoric acid, methane sulfonic acid, benzoic acid, malonic acid, oxalic acid, pyruvic acid, oxalocetic acid (oxobutanic acid), oxal acetic acid ester, 1-hydroxyethane-1,1-diphosphonic acid, as well as the mixtures thereof.

10. Kit-of-parts according to one of the claims 8 or 9, **characterized in that** the second preparation (2) exists in the form of an emulsion.

11. Method for coloration and reductive coloration of keratinous fibers, more particularly human hair, comprising the following method steps in the stated sequence
(a) Application of a cosmetic colorant containing at least one partially oxidizing dye and/or at least one oxidative dye precursor to keratinous fibers
(b) Allowing the colorant to take effect for a period of 5 to 60 minutes
(c) Rinsing out the colorant
(d) Application of a decoloration agent according to one of the claims 1 to 7 or of a decoloration agent produced from a kit-of-parts according to one of the claims 8 to 10 onto keratinous fibers,
(e) Allowing the decoloration agent to take effect at 20 to 45 °C for a period of 5 to 60 minutes, preferably 10 to 55 minutes, more preferably 15 to 55 minutes, most preferably 20 to 50 minutes,
(f) Rinsing out the decoloration agent,
(g) optionally, application of a post-treatment agent to the keratinous fibers, wherein said post-treatment agent contains at least one tenside from the group of anionic tensides, cationic tensides, non-ionic tensides, amphoteric and/or zwitterionic tensides, as well as the mixtures thereof.

12. Use of a cosmetic agent according to one of the claims 1 to 7 or a cosmetic agent produced from a kit-of-parts according to one of the claims 8 to 10 for decoloring dyed keratinous fibers, more particularly human hair.

13. Use of a cosmetic agent according to one of the claims 1 to 7 or a cosmetic agent produced from a kit-of-parts according to one of the claims 8 to 10 for reducing the unpleasant odor produced during the decoloration dyed keratin fibers.

## Revendications

1. Produit cosmétique destiné à la décoloration réductrice de fibres kératiniques colorées, en particulier de cheveux humains, contenant dans un support cosmétique
a) au moins un composé de formule (I)
A[(CR¹R²)SO₂M]_{p,q} (I)
dans laquelle
A représente N(R³)_{3-q} ou O(R⁴)₂₋ₚ
R¹, R², R⁴ représentent indépendamment les uns des autres un atome d'hydrogène ou un groupe alkyle en C₁-C₆,
R³ représente des radicaux identiques ou différents choisis parmi un atome d'hydrogène, un groupe alkyle en C₁-C₂₀, un groupe cycloalkyle en C₃-C₈ éventuellement substitué par un à trois radicaux alkyles en C₁-C₄
M représente des radicaux identiques ou différents choisis parmi un atome d'hydrogène ou un équivalent d'un ion alcalin, alcalino-terreux ou métallique, de préférence un ion sodium, potassium, 1/2 magnésium, 1/2 calcium, 1/2 zinc, ou un ion ammonium (NH₄⁺),
p représente les chiffres 1 ou 2,
q représente les chiffres 1, 2 ou 3,
dans lequel
- au moins un des radicaux R¹, R², R⁴ représente un groupe alkyle en C₁-C₆ lorsque A représente O(R⁴_{2-P}), et
- R³ ne représente pas un atome d'hydrogène lorsque q vaut 1, et
b) au moins un alcool avec de 10 à 30 atomes de carbone en une quantité totale de 0,1 à 20 % en poids par rapport au poids total du produit cosmétique,
**caractérisé en ce qu'**il contient au moins un composé I de formule (la).
N(CH₂SO₂Na)₃ (Ia)

2. Produit cosmétique selon la revendication 1, **caractérisé en ce que** l'au moins un composé I de formule (I) est contenu en une quantité totale de 0,1 à 30,0 % en poids, de préférence de 0,2 à 20,0 % en poids, plus préférablement de 0,3 à 10,0 % en poids, en particulier de 0,5 à 6,0 % en poids par rapport au poids total du produit cosmétique.

3. Produit cosmétique selon l'une des revendications précédentes, **caractérisé en ce que** l'au moins un alcool b) avec de 10 à 30 atomes de carbone est choisi dans le groupe constitué par du 1-décanol, de l'alcool laurylique, de l'alcool myristylique, de l'alcool cétylique, de l'alcool stéarylique, de l'alcool arachidylique, de l'alcool béhénique, de l'alcool lignocérique, de l'alcool cérylique, de l'alcool montanylique et leurs mélanges, en particulier d'un mélange d'alcool cétylique et d'alcool stéarylique.

4. Produit cosmétique selon l'une des revendications précédentes, **caractérisé en ce que** l'au moins un alcool b) est contenu en une quantité totale de 0,5 à 18 % en poids, de préférence de 1,0 à 15 % en poids, plus préférablement de 1,2 à 12 % en poids, en particulier de 1,5 à 10 % en poids par rapport au poids total du produit cosmétique.

5. Produit cosmétique selon l'une des revendications précédentes, **caractérisé en ce qu'**il contient également un composé II de formule (II)
A[(CR¹R²)SO₃M]_{P,q} (II)
dans laquelle
A représente N(R³)_{3-q} ou O(R⁴)₂₋ₚ
R¹, R², R⁴ représentent indépendamment les uns des autres un atome d'hydrogène ou un groupe alkyle en C₁-C₆,
R³ représente des radicaux identiques ou différents choisis parmi un atome d'hydrogène, un groupe alkyle en C₁-C₂₀, un groupe cycloalkyle en C₃-C₈ éventuellement substitué par un à trois radicaux alkyles en C₁-C₄
M représente des radicaux identiques ou différents choisis parmi un atome d'hydrogène ou un équivalent d'un ion alcalin, alcalino-terreux ou métallique, de préférence un ion sodium, potassium, 1/2 magnésium, 1/2 calcium, 1/2 zinc, ou un ion ammonium (NH₄⁺),
p représente les chiffres 1 ou 2,
q représente les chiffres 1, 2 ou 3,
dans lequel
- au moins un des radicaux R¹, R², R⁴ représente un groupe alkyle en C₁-C₆ lorsque A représente O(R⁴₂₋ₚ), et
- R³ ne représente pas un atome d'hydrogène lorsque q vaut 1,
**caractérisé en ce qu'**il contient au moins un composé II de formule (IIa)
N(CH₂SO₃Na)₃ (IIa)

6. Produit cosmétique selon la revendication 5, **caractérisé en ce que** l'au moins un composé II de formule (II) est contenu en une quantité totale de 0,1 à 30,0 % en poids, de préférence de 0,2 à 20,0 % en poids, plus préférablement de 0,3 à 10,0 % en poids, en particulier de 0,5 à 6,0 % en poids par rapport au poids total du produit cosmétique.

7. Produit cosmétique selon l'une des revendications précédentes, **caractérisé en ce qu'**il est présent sous forme d'émulsion.

8. Unité d'emballage à plusieurs composants (assemblage de composants) pour la décoloration réductrice de fibres kératiniques colorées, comprenant au moins deux préparations (1) et (2) confectionnées séparément l'une de l'autre,
- la première préparation (1) contenant dans un support cosmétique au moins un composé I de formule (I) selon la revendication 1 et éventuellement au moins un composé II de formule (II) selon la revendication 5, et
- la seconde préparation (2) contenant au moins un acide inorganique et/ou un acide organique,
la préparation (1) et/ou la préparation (2) contenant au moins un alcool avec de 10 à 30 atomes de carbone en une quantité totale de 0,1 à 20 % en poids sur la base du poids total de la préparation (2).

9. Unité d'emballage à plusieurs composants (assemblage de composants) selon la revendication 8, **caractérisée en ce que** la seconde préparation (2) contient au moins un acide organique choisi dans le groupe constitué par de l'acide citrique, de l'acide tartrique, de l'acide malique, de l'acide lactique, de l'acide acétique, de l'acide sulfurique, de l'acide chlorhydrique, de l'acide phosphorique, de l'acide méthanesulfonique, de l'acide benzoïque, de l'acide malonique, de l'acide oxalique, de l'acide pyruvique, de l'acide oxaloacétique (acide oxobutanedioïque), de l'ester d'acide oxaloacétique, de l'acide 1-hydroxyéthane-1,1-diphosphonique et leurs mélanges.

10. Unité d'emballage à plusieurs composants (assemblage de composants) selon l'une des revendications 8 ou 9, **caractérisée en ce que** la seconde préparation (2) est présente sous forme d'émulsion.

11. Procédé de coloration et de décoloration réductrice de fibres kératiniques, en particulier de cheveux humains, comprenant les étapes de procédé suivantes dans l'ordre indiqué
(a) appliquer un produit de coloration cosmétique contenant au moins un colorant montant directement sur la fibre et/ou au moins un précurseur de colorant d'oxydation sur les fibres kératiniques
(b) laisser agir le produit de coloration pendant une durée de 5 à 60 minutes
(c) rincer le produit de coloration
(d) appliquer un produit de décoloration selon l'une des revendications 1 à 7 ou un produit de décoloration réalisé à partir d'un assemblage de composants selon l'une des revendications 8 à 10 sur les fibres kératiniques,
(e) laisser agir le produit de décoloration à une température de 20 à 45 °C pendant une durée de 5 à 60 minutes, de préférence de 10 à 55 minutes, plus préférablement de 15 à 55 minutes, en particulier de 20 à 50 minutes,
(f) rincer le produit de décoloration,
(g) éventuellement appliquer un produit post-traitement sur les fibres kératiniques, le produit post-traitement contenant au moins un tensioactif dans le groupe constitué par des tensioactifs anioniques, des tensioactifs cationiques, des tensioactifs non ioniques, des tensioactifs amphotères et/ou des tensioactifs zwitterioniques et leurs mélanges.

12. Utilisation d'un produit cosmétique selon l'une des revendications 1 à 7 ou d'un produit cosmétique réalisé à partir d'un assemblage de composants selon l'une des revendications 8 à 10 pour la décoloration des fibres kératiniques colorées, en particulier de cheveux humains.

13. Utilisation d'un produit cosmétique selon l'une des revendications 1 à 7 ou d'un produit cosmétique réalisé à partir d'un assemblage de composants selon l'une des revendications 8 à 10 pour la réduction de la charge olfactive pendant la décoloration de fibres kératiniques colorées.
